(19) Europäisches Patentamt
European Patent Office
Office européen des brevets

(11) Numéro de publication: **0 037 784**
**B1**

# (12) FASCICULE DE BREVET EUROPEEN

(45) Date de publication du fascicule du brevet: **01.08.84**

(21) Numéro de dépôt: **81400538.5**

(22) Date de dépôt: **03.04.81**

(51) Int. Cl.³: **C 07 D 498/06,** A 61 K 31/535 // C07D209/90 ,(C07D498/06, 265/00, 209/00)

(54) **Nouveaux dérivés de l'indolobenzoxazine, leurs sels, procédé de préparation, application à titre de médicaments et compositions les renfermant.**

(30) Priorité: **03.04.80 FR 8007544**

(43) Date de publication de la demande: **14.10.81 Bulletin 81/41**

(45) Mention de la délivrance du brevet: **01.08.84 Bulletin 84/31**

(84) Etats contractants désignés: **AT BE CH DE FR GB IT LI NL SE**

(56) Documents cités:
**EP - A - 0 033 767**

**Burger's Medicinal Chemistry, Part III, edition 4, page 419 (1981)**

(73) Titulaire: **ROUSSEL-UCLAF, 35, boulevard des Invalides, F-75007 Paris (FR)**

(72) Inventeur: **Nedelec, Lucien, 45, boulevard de L'Ouest, F-93340 Le Raincy (FR)**
Inventeur: **Pierdet, André, 10, rue Charles Baudelaire, F-93130 Noisy-le-Sec (FR)**
Inventeur: **Dumont, Claude, 33, rue du Maréchal Vaillant, F-94130 Nogent-sur-Marne (FR)**

(74) Mandataire: **Fritel, Hubert et al, 102, route de Noisy Boîte Postale No 9, F-93230 Romainville (FR)**

**EP 0 037 784 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne des dérivés de l'indolobenzoxazine ainsi que leurs sels, le procédé de préparation, l'application à titre de médicaments de ces dérivés et les compositions les renfermant.

L'invention a pour objet de dérivés de l'indolobenzoxazine, ainsi que leurs sels d'addition avec les acides minéraux ou organiques, caractérisés en ce qu'ils répondent à la formule générale I':

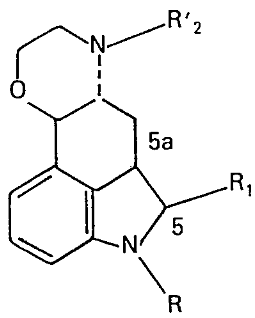

I'

dans laquelle R représente un atome d'hydrogène, un radical alcoyle renfermant de 1 à 5 atomes de carbone, ou un radical aralcoyle renfermant de 7 à 12 atomes de carbone, éventuellement substitué par des radicaux chloro, bromo, méthyl, éthyl, méthoxy, trifluorométhyl ou méthylthio, $R_1$ représente un atome d'hydrogène, de chlore ou de brome, $R'_2$ représente un atome d'hydrogène, un radical alcoyle renfermant de 1 à 5 atomes de carbone, éventuellement substitué par un atome d'halogène ou par un radical hydroxy, $R'_2$ représente encore un radical cycloalcoylalcoyle renfermant de 4 à 7 atomes de carbone, alcényle ou alcynyle renfermant de 3 à 7 atomes de carbone, ou aralcoyle renfermant de 7 à 12 atomes de carbone, éventuellement substitué par des radicaux chloro, bromo, méthyl, éthyl, méthoxy, trifluorométhyl ou méthylthio.

L'invention a notamment pour objet, parmi les dérivés de l'indolobenzoxazine de formule I', ceux répondant à la formule I:

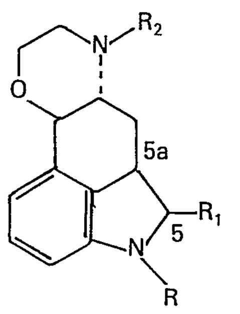

I

dans laquelle R et $R_1$ sont définis comme précédemment et $R_2$ représente un atome d'hydrogène, un radical alcoyle renfermant de 1 à 5 atomes de carbone, cycloalcoylalcoyle renfermant de 4 à 7 atomes de carbone, alcényle ou alcynyle renfermant de 3 à 7 atomes de carbone, ou aralcoyle renfermant de 7 à 12 atomes de carbone, éventuellement substitué par des radicaux chloro, bromo, méthyl, éthyl, méthoxy, trifluorométhyl ou méthylthio.

Dans les formules générales I' et I et dans ce qui suit, le terme radical alcoyle renfermant de 1 à 5 atomes de carbone désigne, par exemple, un radical méthyl, éthyl, propyl, isopropyl, butyl, isobutyl ou pentyl; le terme radical aralcoyle renfermant de 7 à 12 atomes de carbone désigne, par exemple, un radical benzyl ou phénéthyl; le terme radical cycloalcoylalcoyle renfermant de 4 à 7 atomes de carbone désigne, par exemple, un radical cyclopropyl méthyl; le terme radical alcényle renfermant de 3 à 7 atomes de carbone désigne, par exemple, un radical allyl ou buten-2-yl; le terme radical alcynyle renfermant de 3 à 7 atomes de carbone désigne, par exemple, un radical propargyl.

Les sels d'addition avec les acides minéraux ou organiques peuvent être, par exemple, les sels formés avec les acides chlorhydrique, bromhydrique, iodhydrique, nitrique, sulfurique, phosphorique, acétique, formique, propionique, benzoïque, maléique, fumarique, succinique, tartrique, citrique, oxalique, glyoxylique, aspartique, alcanesulfoniques, tels que les acides méthane et éthansulfoniques, arylsulfoniques, tels que les acides benzène et paratoluènesulfoniques et arylcarboxyliques.

La liaison portée en pointillé dans le cycle morpholino signifie que la jonction entre le cycle morpholino et le cycle cyclohexane est trans. Bien entendu, le racémique, aussi bien que les isomères optiquement actifs correspondants entrent dans le cadre de l'invention.

Parmi les produits, objet de l'invention, on peut citer notamment les dérivés répondant à la formule I ci-dessus, ainsi que leurs sels d'addition avec les acides minéraux ou organiques, caractérisés en ce que, dans ladite formule (I), R représente un atome d'hydrogène et $R_2$ représente un atome d'hydrogène ou un radical alcoyle renfermant de 1 à 5 atomes de carbone ou cycloalcoylalcoyle renfermant de 4 à 7 atomes de carbone.

Parmi ceux-ci, on peut citer plus particulièrement les dérivés caractérisés en ce que dans ladite formule (I), $R_1$ représente un atome d'hydrogène ou de brome, et $R_2$ représente un atome d'hydrogène ou un radical alcoyle renfermant de 1 à 5 atomes de carbone, ainsi que leurs sels d'addition avec les acides minéraux ou organiques et tout particulièrement les dérivés décrits dans les exemples.

L'invention a également pour objet un procédé de préparation des dérivés, tels que définis par la formule (I') ci-dessus, ainsi que de leurs sels, caractérises en ce que l'on réduit un produit de formule II:

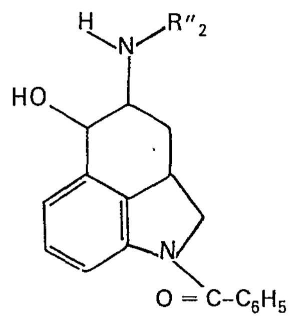

II

dans laquelle $R''_2$ représente un atome d'hydrogène, un radical alcoyle renfermant de 1 à 5 atomes de carbone, cycloalcoylalcoyle renfermant de 4 à 7 atomes de carbone, ou aralcoyle renfermant de 7 à 12 atomes de carbone, éventuellement substitué, pour obtenir un produit de formule III:

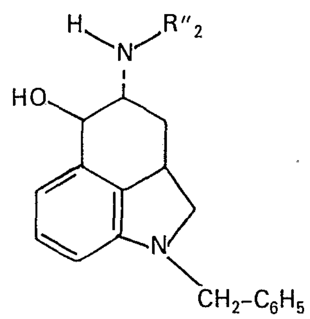

III

dans laquelle $R''_2$ a la signification déjà indiquée, que l'on fait réagir avec le chlorure de chloroacétyle pour obtenir un produit de formule IV:

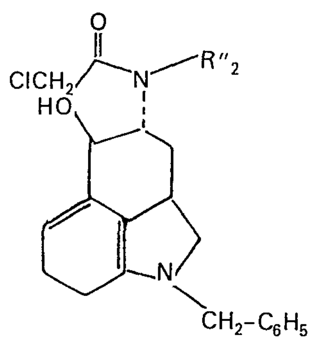

IV

dans laquelle $R''_2$ a la signification déjà indiquée, que l'on cyclise pour obtenir un produit de formule V:

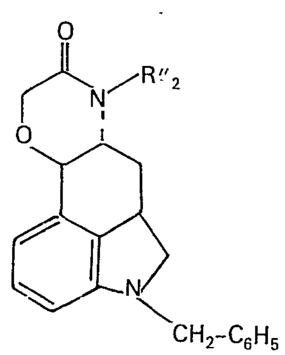

V

dans laquelle $R''_2$ a la signification déjà indiquée, que l'on réduit pour obtenir un produit de formule $I'_A$:

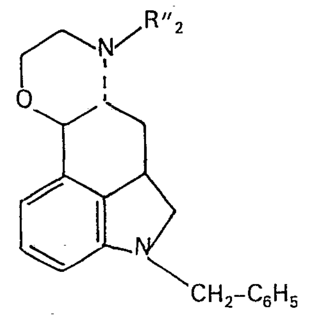

$I'_A$

dans laquelle $R''_2$ a la signification déjà indiquée, correspondant à un produit de formule I', dans laquelle $R'_2$ à la signification de $R''_2$, $R_1$ représente un atome d'hydrogène et R représente un radical benzyle, produit de formule $I'_A$ que, le cas échéant,

a) soit dans le cas où $R''_2$ représente un atome d'hydrogène, l'on fait réagir avec un halogénure de formule VI:

Hal-$R'''_2$ VI

dans laquelle Hal représente un atome de chlore, de brome ou d'iode, et $R'''_2$ a la signification de $R'_2$ déjà indiquée à l'exception de l'hydrogène et d'halogénoalcoyle, pour obtenir un produit de formule $I'_B$:

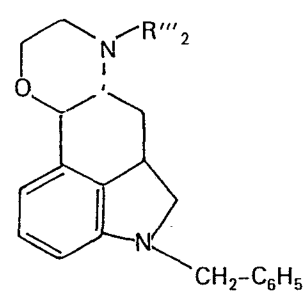

$I'_B$

dans laquelle $R'''_2$ a la signification déjà indiquée, correspondant à un produit de formule I' dans laquelle $R'_2$ a la signification de $R'''_2$, $R_1$ représente un atome d'hydrogène et R représente un radical benzyle, produit de formule $I'_B$ que, si désiré, dans le cas ou $R'''_2$ représente un radical hydroxyalcoyle, l'on traite par un agent d'halogénation pour obtenir un produit de formule $I''_B$:

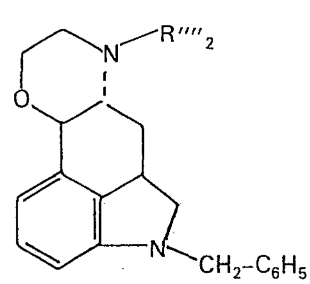

$I''_B$

dans laquelle R""$_2$ représente un radical halogénoalcoyle, correspondant à un produit de formule I' dans laquelle R'$_2$ a la signification de R""$_2$, $R_1$ représente un atome d'hydrogène, R représente un radical benzyle;

b) soit, dans le cas où R'$_2$ représente un atome d'hydrogène, l'on fait agir avec un agent de méthylation, pour obtenir un produit de formule I'$_C$:

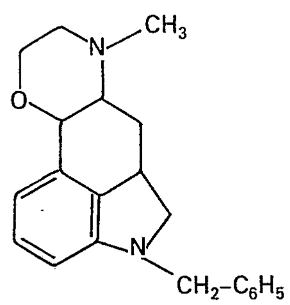

I'$_C$

correspondant à un produit de formule I' dans laquelle R'$_2$ représente un radical méthyle, $R_1$ représente un atome d'hydrogène et R représente un radical benzyle, produits de formule I'$_A$, I'$_B$, I"$_B$ et I'$_C$ que, si désiré, l'on hydrogènolyse, pour obtenir un produit de formule I'$_D$:

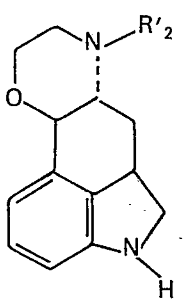

I'$_D$

dans laquelle R'$_2$ a la signification déjà indiquée, correspondant à la signification du substituant de l'atome d'azote dans les produits de formule I'$_A$, I'$_B$, I"$_B$ et I'$_C$, produit de formule I'$_D$ correspondant à un produit de formule I' dans laquelle R'$_2$ à la valeur indiquée ci-dessus et R et $R_1$ représentent un atome d'hydrogène, produit de formule I'$_D$ que, si désiré,

- soit l'on isole,
- soit l'on fait réagir avec un halogénure de formule VII:

Hal-R' VII

dans laquelle Hal représente un atome de chlore, de brome ou d'iode et R' représente un radical alcoyle renfermant de 1 à 5 atomes de carbone ou un radical aralcoyle renfermant de 7 à 12 atomes de carbone, éventuellement substitué, pour obtenir un produit de formule I"$_D$:

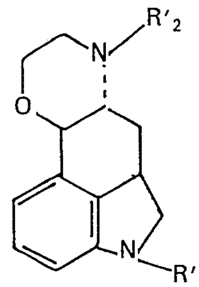

I"$_D$

dans laquelle R' et R'$_2$ ont la signification déjà indiquée, correspondant à un produit de formule I' dans laquelle R'$_2$ a la valeur indiquée ci-dessus, $R_1$ représente un atome d'hydrogène et R a la valeur de R', puis, si désiré, salifie les produits de formule I'$_A$, I'$_B$, I'$_C$, I'$_C$ I'$_D$, I"$_D$.

La réduction du produit de formule II est réalisée de préférence à l'aide d'un hydrure métallique, tout particulièrement à l'aide d'hydrure d'aluminium-lithium en présence d'un acide de Lewis, notamment le chlorure d'aluminium. Pour mener à bien cette réduction, on peut utiliser un solvant tel que le diméthoxyéthane ou d'autres éthers tels que le tétrahydrofurane ou le dioxane, de préférence ce dernier.

La condensation du produit de formule III avec le chlorure de chloroacétyle est avantageusement effectuée en présence d'un agent fixateur d'acides, de préférence l'hydroxyde de sodium, dans un solvant organique tel que le dioxane ou le tétrahydrofurane, mais de préférence dans le chloroforme.

La cyclisation du produit de formule IV est effectué par exemple à l'aide d'une base forte telle qu'un hydroxyde alcalin, comme l'hydroxyde de sodium ou de potassium, un alcoolate alcalin comme l'éthylate ou le terbutylate de sodium, mais de préférence à l'aide d'hydrure de sodium; elle est avantageusement réalisée dans un éther tel que le dioxane ou le tétrahydrofurane, mais de préférence dans le diméthoxyéthane.

La réduction du produit de formule V est réalisée de préférence à l'aide d'un hydrure métallique particulièrement à l'aide d'hydrure d'aluminium lithium dans un éther, notamment au reflux du tétrahydrofurane.

L'halogénure de formule VI peut être un chlorure ou un bromure, mais de préférence un iodure. Il réagit avantageusement avec l'amine secondaire de formule (I'$_A$), dans laquelle R"$_2$ représente l'hydrogène, en présence d'un agent fixateur d'acide, comme par exemple un carbonate alcalin tel que le carbonate de potassium, dans un solvant tel que le diméthylformamide.

L'agent d'halogénation que l'on fait agir sur le produit de formule I'$_B$ est de préférence un agent de chloration tel que le chlorure de méthane sulfonyle.

La méthylation conduisant au produit de formule I'$_C$ peut être réalisée à l'aide d'un mélange

d'aldéhyde formique et d'acide formique, mais on utilise de préférence l'aldéhyde formique en présence d'un réducteur tel que le cyano borhydrure de sodium. Le solvant utilisé est avantageusement l'acétonitrile.

L'hydrogénolyse des produits de formule $I'_A$, $I'_B$, $I''_B$ ou $I'_C$ est réalisé de préférence par action de l'hydrogène en présence d'un catalyseur tel que le Nickel de Raney, le carbonate de strontium palladié ou l'hydroxyde de palladium, mais plus particulièrement en présence de palladium sur du charbon ou du talc.

L'agent d'halogénation que l'on fait agir sur le composé de formule $I'_D$ ou $I'_E$ peut être, par exemple, la N-chloro succinimiode dans le cas de la chloration, la N-bromosuccinimide ou de préférence le complexe bromé de la pyrrolidone de formule:

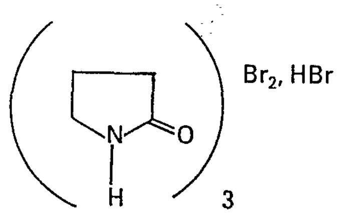

dans le cas de la bromation.

L'halogénure de formule VII est de préférence un iodure. On le fait avantageusement réagir avec le produit de formule $I'_E$ en présence d'un agent fixateur d'acides tel qu'un carbonate ou un hydroxyde alcalin, le carbonate de sodium ou l'hydroxyde de potassium par exemple.

Dans les formules II, III et IV, le trait pointillé signifie que les substituants hydroxyles et amino sont en position trans.

Les dérivés de formule I' présentent un caractère basique. On peut avantageusement préparer les sels d'addition des dérivés de formule (I'), en faisant réagir, en proportions sensiblement stoechiométriques, un acide minéral ou organique avec ledit dérivé de formule (I'). Les sels peuvent être préparés sans isoler les bases correspondantes.

Les racémates de formule (I') peuvent être résolus en leurs énantiomères optiquement actifs par des méthodes connues en elles-mêmes, telles que, par exemple, la formation de sels au moyen d'acides optiquement actifs.

Les dérivés, objet de la présente invention, possèdent de très intéressantes propriétés pharmacologiques; ils sont doués notamment de remarquables propriétés agonistes dopaminergiques, ainsi que des propriétés hypotensives et anti-hypertensives.

Ces propriétés pharmacologiques sont illustrées plus loin dans la partie expérimentale.

Ces propriétés justifient l'utilisation des dérivés de l'indolobenzoxazine, ainsi que de leurs sels pharmaceutiquement acceptables, à titre de médicaments.

La présente demande a ainsi également pour objet l'application, à titre de médicaments, des dérivés de l'indolobenzoxazine, tels que définis par la formule I', et notamment des dérivés tels que définis par la formule (I), ainsi que de leurs sels d'addition avec les acides pharmaceutiquement acceptables.

Parmi les médicaments, selon l'invention, on retient, de préférence, les médicaments caractérisés en ce qu'ils sont constitués par les nouveaux dérivés de l'indolobenzoxazine, répondant à la formule (I), dans laquelle R représente un atome d'hydrogène et $R_2$ représente un atome d'hydrogène ou un radical alcoyle renfermant de 1 à 5 atomes de carbone ou cycloalcoylalcoyle renfermant de 4 à 7 atomes de carbone, ainsi que par leurs sels d'addition avec les acides pharmaceutiquement acceptables.

Parmi ceux-ci, on retient notamment ceux répondant à la formule (I), dans laquelle $R_1$ représente un atome d'hydrogène ou de brome et $R_2$ représente un atome d'hydrogène ou un radical alcoyle renfermant de 1 à 5 atomes de carbone, ainsi que leurs sels d'addition avec les acides pharmaceutiquement acceptables.

Parmi ces derniers, on retient tout particulièrement les dérivés cités dans les exemples.

Les médicaments selon l'invention trouvent leur emploi par exemple dans le traitement des syndromes neurologiques d'origine extrapyramidale, par exemple dans le traitement de la maladie de Parkinson et dans le traitement des syndromes parkinsonniens post encéphalitiques; ils peuvent également être utilisés dans le traitement de l'hypersécrétion prolactine par l'antéhypophyse, par exemple dans le traitement de l'hypogonadisme de la femme ou de l'homme. Ils peuvent aussi être utilisés dans le traitement de la senescence cérébrale ou des manifestations liées à une hypoxie cérébrale.

En raison de leurs propriétés hypotensives et antihypertensives, ils peuvent également être utilisés dans le traitement de l'hypertension artérielle essentielle, de l'hypertension de la cinquantaine, de la ménopause, du diabétique, de l'obèse et du pléthorique, ainsi que dans le traitement de l'hypertension artérielle du sujet agé, de l'athérosclérose et dans le traitement de l'hypertension d'origine rénale.

La dose usuelle, variable selon le dérivé utilisé, le sujet traité et l'affection en cause peut être, par exemple, de 0,1 mg à 50 mg par jour, par voie orale chez l'homme pour le traitement de la maladie de Parkinson.

L'invention a enfin pour objet les compositions pharmaceutiques qui renferment au moins un dérivé précité ou l'un de ses sels d'addition avec les acides pharmaceutiquement acceptables, à titre de principe actif.

A titre de médicaments, les dérivés répondant à la formule I' et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme, par exemple, les comprimés, simples ou dragéifiés, les gélules, les granulés, les suppositoires, les préparations injectables; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les dérivés de formule II peuvent être préparés comme suit: on effectue un réarrangement de Neber [Chem. Rev. 64, 81 (1964)] sur la tosyloxime de formule VIII:

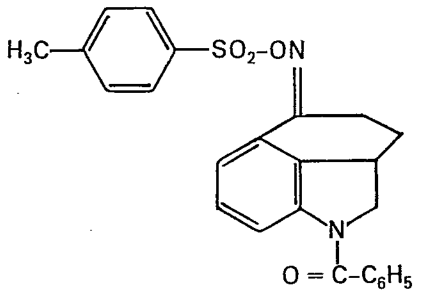

VIII

décrite dans J. Am. Soc. Vol 78 (1965), p. 3087 et suivantes, réduit l'aminocétone ainsi obtenue, par exemple à l'aide de borohydrure de sodium, pour obtenir un dérivé de formule IX:

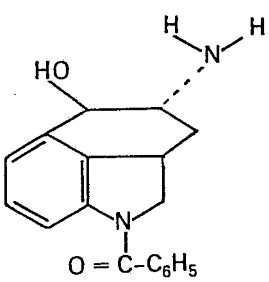

IX

correspondant à un produit de formule II dans laquelle $R''_2$ représente l'hydrogène, que l'on fait réagir si désiré avec l'anhydride trifluoroacétique, de préférence en présence d'une base telle qu'un hydroxyde alcalin, pour obtenir un produit de formule X:

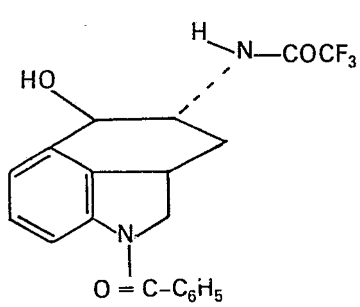

X

que l'on fait réagir en présence d'un agent alcalin tel qu'un carbonate ou un hydroxyde alcalin, ou une amine tertiaire, avec un halogénure de formule XI:

$$Hal{-}R_3 \qquad XI$$

dans laquelle Hal a la signification déjà indiquée, et $R_3$ a la signification de $R''_2$ déjà indiquée, à l'exception de l'hydrogène, pour obtenir un produit de formule XII:

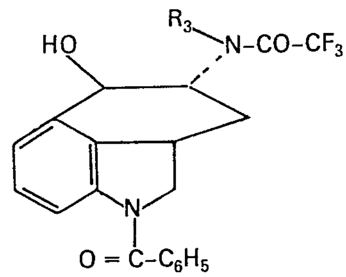

XII

dans laquelle $R_3$ a la signification déjà indiquée, que l'on soumet à un agent capable d'hydrolyser sélectivement l'amide trifluoroacétique, tel que le carbonate de sodium, pour obtenir le produit de formule II cherché.

Un exemple de préparation d'un produit de formule II dans laquelle $R''_2$ représente un atome d'hydrogène figure ci-après dans la partie expérimentale.

Il va être donné maintenant, à titre non limitatif, des exemples de mise en œuvre de l'invention.

Exemple 1

5, 5a, 6, 6a (RS), 7, 8, 9, 10a (RS)- octahydro 4-phénylméthyl 7H-indolo [3,4-g, h] [1,4] benzoxazine.

Stade A

4-amino 1, 2, 2a, 3, 4 (RS), 5 (RS)-hexahydro 1-phénylméthyl benz [c,d] indol-5-ol.

On ajoute lentement sous atmosphère inerte et agitation 26 g de 4-amino 1-benzoyl 1, 2, 2a, 3, 4 (RS), 5 (RS)-hexahydro benz [c,d] indol-5-ol, à 26 g d'hydrure d'aluminium lithium et 13 g de chlorure d'aluminium dans 800 cm³ de dioxane, porte 2 heures au reflux, refroidit à 0, +5°C, ajoute goutte à goutte 300 cm³ de tétrahydrofurane hydraté à 20% et 300 cm³ de soude 2N, essore le précipité formé, lave au chlorure de méthylène, reprend le filtrat par 1,5 l de chlorure de méthylène, lave à l'eau, sèche, évapore à sec et récupère 20 g du produit attendu fondant à 166°C après recristallisation dans le chlorure de méthylène.

Stade B

2-chloro N-[5-hydroxy 1-phénylméthyl 1, 2, 2a, 3, 4 (RS)-hexahydro 4-benz [c,d] indolyl] acétamide.

On agite 26 g de produit tel qu'obtenu au stade précédent dans 800 cm³ de chloroforme, ajoute 26 g de soude en solution dans 200 cm³ d'eau, puis goutte à goutte 14,8 cm³ de chlorure de chloroacétyle, après 1 heure 30 minutes ajoute de l'eau, extrait au chloroforme, lave à l'eau, sèche, amène à sec sous pression réduite, ajoute quelques $cm^3$ d'éther, essore, sèche sous pression réduite et obtient 29,5 g de produit attendu fondant à 211°C.

Stade C

4, 5, 5a, 6, 6a (RS), 7, 9, 10a (RS)-octahydro 4-phénylméthyl 8H-indolo [3,4-gh] [1,4] benzoxazin-8-on.

On ajoute lentement sous agitation et atmosphère inerte une solution tiède de 356 mg de produit obtenu au stade précédent dans 35 $cm^3$ de diméthoxyéthane, à 27,5 mg d'hydrure de sodium et 2 $cm^3$ de diméthoxyéthane, après 1 heure verse dans 50 $cm^3$ d'eau, extrait au chlorure de méthylène, lave à l'eau, sèche, évapore à sec sous pression réduite et obtient 260 mg de produit attendu fondant à 260°C.

Stade D

5, 5a, 6, 6a (RS), 7, 8, 9, 10a (RS) octahydro 4-phénylméthyl 7H-indolo [3,4-g, h] [1,4] benz oxazine.

On agite sous atmosphère inerte 1 g d'hydrure d'aluminium-lithium dans 100 $cm^3$ de tétrahydrofurane, ajoute peu a peu 1 g de produit tel qu'obtenu au stade précédent, chauffe 1 heure au reflux, refroidit par un bain de glace-méthanol, ajoute goutte à goutte 50 $cm^3$ de tétrahydrofurane hydraté à 20%, filtre, lave au tétrahydrofurane puis au chlorure de méthylène, décante le filtrat, lave à l'eau, sèche, évapore à sec sous pression réduite, purifie par chromatographie sur silice (Eluant: chloroforme-méthanol 95-5) la résine obtenue, récupère les fractions de Rf = 0,3 et obtient 684 mg de produit attendu, fondant à 130°C après recristallisation dans l'éther éthylique.

Exemple 2

5, 5a, 6, 6a (RS), 7, 8, 9, 10a (RS)-octahydro 4H-indolo [3,4-g,h] [1,4] benzoxazine.

On agite sous arrivée d'hydrogène 8 g de produit tel qu'obtenu à l'exemple I, avec 100 $cm^3$ d'acide acétique pur en présence de 2 g de carbone à 10% de palladium, après 1 heure 30 minutes filtre, rince à l'acide acétique, amène à sec sous pression réduite, ajoute 250 $cm^3$ d'eau, refroidit, alcalinise par de la lessive de soude, extrait au chlorure de méthylène, lave à l'eau, sèche, amène à sec sous pression réduite, purifie par chromatographie sur silice (Eluant: chloroforme-méthanol 95-5) les 5,4 g de résine obtenus, récupère les fractions de Rf = 0,2 et obtient 4,75 g de produit attendu qui après recristallisation dans le méthanol fond à 128°C et 158°C.

Le 4-amino 1-benzoyl 1, 2, 2a, 3, 4 (RS), 5 (RS)-hexahydro benz [c, d] indol-5-ol de départ peut être préparé comme suit: On prépare de l'éthylate de potassium à partir de 20 $cm^3$ d'éthanol et 430 mg de potassium à 98%, refroidit à 0°C, ajoute une solution de 4,46 g de [(4-méthylphényl) sulfonyl] oxime de 1, 2, 2a, 3, 4 (RS), 5 (RS)-hexahydro benz [c, d] indol-5-one dans 20 $cm^3$ d'éthanol et 20 $cm^3$ de chloroforme, laisse une nuit sous agitation à 0°C, amène à sec sous pression réduite, ajoute 200 $cm^3$ d'éther, extrait à l'aide d'une solution aqueuse d'acide chlorhydrique 2N, décante, évapore sous pression réduite la phase aqueuse, reprend plusieurs fois à l'éthanol et amène à sec sous vide. On ajoute goutte à goutte une solution de 1,25 g de borohydrure de sodium à 95% dans 25 $cm^3$ d'éthanol, sous agitation, aux 3,8 g de chlorhydrate de 4-amino 1-benzoyl 1, 2, 2a, 3, 4 (RS) 5 (RS)-hexahydro benz [c, d] indol-5-one obtenus ci-dessus dans 80 $cm^3$ d'éthanol à la température de 0°C, après 1 heure laisse remonter la température à 22°C, après une nouvelle heure ajoute quelques $cm^3$ d'eau puis 25 $cm^3$ de soude 2N, extrait au chloroforme, lave à l'eau, sèche, amène à sec sous pression réduite et obtient 1,8 g de produit attendu fondant à 173°C après recristallisation dans le chlorure de méthylène.

Exemple 3

5, 5a, 6, 6a (RS), 7, 8, 9, 10a (RS)-octahydro 4-phénylméthyl 7-méthyl 4H-indolo [3,4-g, h] [1,4] benzoxazine.

On dissout sous agitation et atmosphère inerte 10 g de produit tel qu'obtenu à l'exemple 1 dans 200 $cm^3$ d'acétonitrile vers 50°C, refroidit, ajoute lentement 19,6 $cm^3$ d'une solution aqueuse d'aldéhyde formique à 30%, puis 3,27 g de cyanoborohydrure de sodium, agite pendant 1 heure, évapore sous pression réduite le solvant, ajoute 500 $cm^3$ d'eau, extrait au chlorure de méthylène, lave à l'eau, sèche, amène à sec sous pression réduite, purifie par chromatographie sur silice (Eluant: chloroforme-méthanol 95-5) les 10,2 g de résine obtenus, récupère les fractions de Rf = 0,5 et obtient 9 g de produit attendu fondant à 106°C après recristallisation dans l'éther isopropylique.

Exemple 4

5, 5a, 6, 6a (RS), 7, 8, 9, 10a (RS)-octahydro 7-méthyl 4H-indolo [3,4-g, h] [1,4] benzoxazine.

On agite sous arrivée d'hydrogène les 9 g du produit obtenus à l'exemple 3, dans 150 $cm^3$ d'acide acétique en présence de 2,25 g de carbone à 10% de palladium, après 1 heure 20 minutes filtre, rince plusieurs fois à l'acide acétique, amène à sec sous pression réduite, ajoute de l'eau, refroidit, alcalinise par de la lessive de soude, extrait au chlorure de méthylène, lave à l'eau, sèche, évapore à sec sous pression réduite, purifie par chromatographie sur silice (Eluant: chloroforme-méthanol 95-5) les 7 g de résine obtenus, récupère les fractions de Rf = 0,3 et obtient 4,9 g de produit attendu fondant après recristallisation dans l'éther isopropylique à 135°C puis 148°C.

Exemple 5

5, 5a, 6, 6a (RS), 7, 8, 9, 10a (RS)-octahydro 4-phénylméthyl 7-propyl 4H-indolo [3,4-g, h] [1,4] benzoxazine.

On dissout sous agitation et atmosphère inerte 10 g de produit tel qu'obtenu à l'exemple 1 dans

500 cm³ de diméthylformamide, ajoute 9,11 g de carbonate de potassium et 14,2 cm³ d'iodure de propyle, laisse sous agitation pendant 3 heures à 50°C, refroidit, verse dans 1,5 l d'eau, extrait à l'éther, lave à l'eau, sèche, amène à sec sous pression réduite, purifie par chromatographie sur silice (Eluant: benzène-acétate d'éthyle 5-5) et obtient 8,9 g de produit attendu fondant à 105°C après recristallisation dans l'éther isopropylique.

Exemple 6

5, 5a, 6, 6a (RS), 7, 8, 9, 10a (RS)-octahydro 7-propyl 4H-indolo [3,4-g, h] [1,4] benzoxazine.

On agite sous courant d'hydrogène 8,75 g du produit obtenu à l'exemple 5 dans 100 cm³ d'acide acétique en présence de 2,2 g de carbone à 10% de palladium, après 1 heure et 30 minutes, filtre, rince à l'acide acétique, amène à sec sous pression réduite, ajoute 150 cm³ d'eau, refroidit, alcalinise par de la lessive de soude, extrait au chlorure de méthylène, lave à l'eau, sèche, amène à sec sous pression réduite, purifie par chromatographie sur silice (Eluant: chloroforme-méthanol 95-5) les 6,5 g de résine obtenus, récupère les fractions de Rf = 0,3 et obtient 5,4 g de produit attendu fondant à 85°C après recristallisation dans l'éther isopropylique.

Exemple 7

5, 5a, 6, 6a (RS), 7, 8, 9, 10a (RS)-octahydro 4-phényl méthyl 7-(2-hydroxy)éthyl 4H-indolo [3,4-g, h] [1,4] benzoxazine.

On dissout sous agitation et atmosphère inerte 19 g de produit tel qu'obtenu à l'exemple 1, dans 350 cm³ de diméthylformamide et ajoute 17 g de carbonate de potassium. On ajoute ensuite 9,2 cm³ de bromoéthanol puis maintient à 50°C sous agitation pendant 2 heures. On refroidit, ajoute de l'eau et extrait au chlorure de méthylène. On lave la phase organique à l'eau, sèche et évapore le solvant. On chromatographie le résidu sur silice en éluant au mélange chloroforme-méthanol (92-2) et obtient 13 g de produit attendu que l'on recristallise dans l'éther isopropylique. F = 108°C.

Exemple 8

5, 5a, 6, 6a (RS), 7, 8, 9, 10a (RS)-octahydro 4-phényl méthyl 7-(2-chloro) éthyl 4H-indolo [3,4-g, h] [1,4] benzoxazine.

On mélange 11,5 g de produit obtenu à l'exemple 7 avec 100 cm³ de pyridine puis ajoute lentement 7 cm³ de chlorure de méthane sulfonyle. On maintient pendant 17 heures à température ambiante, verse dans un mélange eau-glace, alcalinise par addition d'ammoniaque, extrait au chlorure de méthylène, lave la phase organique à l'eau, sèche et évapore le solvant. On chromatographie le résidu sur silice en éluant au mélange chloroforme-méthanol (95-5) et obtient 9,7 g de produit attendu. F = 112°C.

Exemple 9

5, 5a, 6, 6a (RS), 7, 8, 9, 10a (RS)-octahydro 7-(2-chloro) éthyl 4H-indolo [3,4-g, h] [1,4] benzoxazine.

On agite sous courant d'hydrogène, 0,37 g de produit obtenue à l'exemple 8, 10 cm³ d'acide acétique et 0,9 g de carbone à 10% de palladium. Après 3 heures, on filtre le mélange, lave le filtre à l'acide acétique et évapore à sec sous pression réduite. On ajoute de l'eau au résidu, alcalinise par addition d'une solution aqueuse 1N d'hydroxyde de sodium, extrait au chlorure de méthylène, lave la phase organique à l'eau, sèche et évapore le solvant. On chromatographie le résidu sur silice en éluant au mélange chloroforme-méthanol (95-5) et obtient 0,2 g de produit attendu. F = 112°C.

Etude pharmacologique

1) Etude de la toxicité aiguë

On a évalué la dose létale $DL_0$ du dérivé de l'exemple 6, après administration par voie intrapéritonéale chez la souris.

On appelle $DL_0$ la dose maximal ne provoquant aucune mortalité.

Le résultat obtenu est le suivant: la $DL_0$ du produit de l'exemple 6 est de 100 mg/kg.

2) Détermination de l'activité hypotensive

L'activité hypotensive a été étudiée sur des rats mâles de souche WISTAR pesant 300 g environ et anesthésiés au nembutal (50 mg/kg par voie intraveineuse).

Le produit testé a été administré par voie intraveineuse dans la veine jugulaire.

La pression artérielle carotidienne a été mesurée avant et après administration du produit testé.

Le tableau ci-après indique les variations exprimées en pourcentage de la pression artérielle après administration du produit testé par rapport à la pression artérielle témoin initiale.

| Produit de l'exemple | Dose mg/kg | Variation % de la pression artérielle | | | |
|---|---|---|---|---|---|
| | | 1 minute après l'administration | 5 minutes après l'administration | 10 minutes après l'administraton | 30 minutes après l'administration |
| 6 | 0,1 | - 44 | - 50 | - 38 | - 30 |
| | 0,01 | - 37 | - 28 | - 24 | - 3 |

**Revendications**

Pour les Etats contractants: BE-CH-DE, FR, GB, IT, LI, NL, SE

1. Dérivés de l'indolobenzoxazine, ainsi que leurs sels d'addition avec les acides minéraux ou organique, caractérisés en ce qu'ils répondent à la formule générale I':

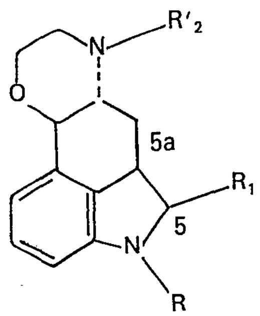

I'

dans laquelle R représente un atome d'hydrogène, un radical alcoyle renfermant de 1 à 5 atomes de carbone, ou un radical aralcoyle renfermant de 7 à 12 atomes de carbone, éventuellement substitué par des radicaux chloro, bromo, méthyl, éthyl, méthoxy, trifluorométhyl on méthylthio, $R^1$ représente un atome d'hydrogène, de chlore ou de brome, $R'_2$ représente un atome d'hydrogène, un radical alcoyle renfermant de 1 à 5 atomes de carbone, éventuellement substitué par un atome d'halogène ou par un radical hydroxy, $R'_2$ représente encore un radical cycloalcoylalcoyle renfermant de 4 à 7 atomes de carbone, alcényle ou alcynyle renfermant de 3 à 7 atomes de carbone, ou aralcoyle renfermant de 7 à 12 atomes de carbone, éventuellement substitué par des radicaux chloro, bromo, méthyl, éthyl, méthoxy, trifluorométhyl ou méthylthio.

2. Dérivés de l'indolobenzoxazine selon la revendication 1, ainsi que leurs sels d'addition avec les acides minéraux ou organiques, caractérisés en ce qu'ils répondent à la formule générale I:

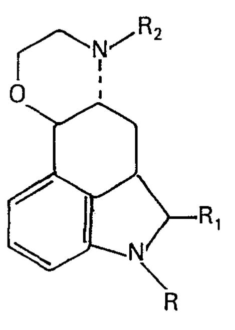

I

dans laquelle R et $R_1$ sont définis comme à la revendication 1 et $R_2$ représente un atome d'hydrogène, un radical alcoyle renfermant de 1 à 5 atomes de carbone, cycloalcoylalcoyle renfermant de 4 à 7 atomes de carbone, alcényle ou alcynyle renfermant de 3 à 7 atomes de carbone, ou aralcoyle renfermant de 7 à 12 atomes de carbone, éventuellement substituée par des radicaux chloro, bromo, méthyl, éthyl, méthoxy, trifluorométhyl ou méthylthio.

3. Dérivés de l'indolobenzoxazine selon la revendication 2, ainsi que leurs sels d'addition avec les acides minéraux ou organiques, caractérisés en ce que R représente un atome d'hydrogène et $R_2$ représente un atome d'hydrogène ou un radical alcoyle renfermant de 1 à 5 atomes de carbone ou cycloalcoylalcoyle renfermant de 4 à 7 atomes de carbone.

4. Dérivés de l'indolobenzoxazine selon la revendication 2, ainsi que leurs sels d'addition avec les acides minéraux ou organiques, caractérisés en ce que R représente un atome d'hydrogène, $R_1$ représente un atome d'hydrogène ou de brome, et $R_2$ représente un atome d'hydrogène ou un radical alcoyle renfermant de 1 à 5 atomes de carbone.

5. La 5, 5a, 6, 6a (RS), 7, 8, 9, 10a (RS)-octahydro 7-propyl 4H-indolo [3,4-g, h] [1,4] benzoxazine, ainsi que ses sels d'addition avec les acides minéraux ou organiques.

6. Procédé de préparation des dérivés, tels que définis par la formule I' de la revendication 1, ainsi que de leurs sels, caractérisé en ce que l'on réduit un produit de formule II:

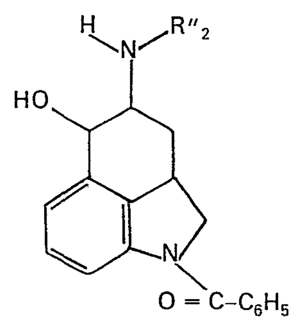

II

dans laquelle $R''_2$ représente un atome d'hydrogène, un radical alcoyle renfermant de 1 à 5 atomes de carbone, cycloalcoylalcoyle renfermant de 4 à 7 atomes de carbone, ou aralcoyle renfermant de 7 à 12 atomes de carbone, éventuellement substitué, pour obtenir un produit de formule III:

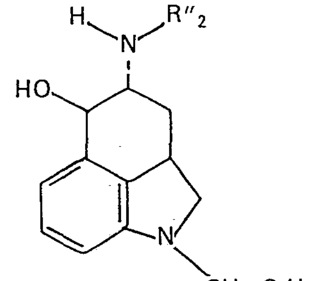

III

dans laquelle $R''_2$ a la signification déjà indiquée, que l'on fait réagir avec le chlorure de chloroacétyle pour obtenir un produit de formule IV:

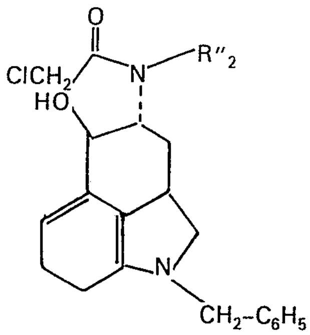

IV

dans laquelle $R''_2$ a la signification déjà indiquée, que l'on cyclise pour obtenir un produit de formule V:

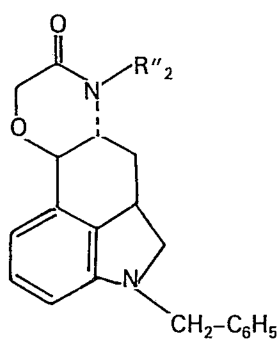

V

dans laquelle $R''_2$ a la signification déjà indiquée, que l'on réduit pour obtenir un produit de formule $I'_A$:

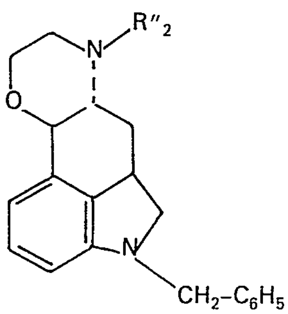

$I'_A$

dans laquelle $R''_2$ a la signification déjà indiquée, correspondant à un produit de formule I', dans laquelle $R'_2$ à la signification de $R''_2$, $R_1$ représente un atome d'hydrogène et R représente un radical benzyle, produit de formule $I'_A$ que, le cas échéant,

a) soit dans le cas où $R''_2$ représente un atome d'hydrogène, l'on fait réagir avec un halogénure de formule VI:

Hal-$R'''_2$ VI

dans laquelle Hal représente une atome de chlore, de brome ou d'iode, et $R'''_2$ a la signification de $R'_2$ déjà indiquée à l'exception de l'hydrogène et d'halogénoalcoyle, pour obtenir un produit de formule $I'_B$:

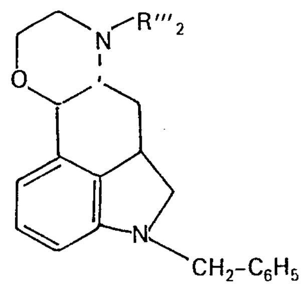

$I'_B$

dans laquelle $R'''_2$ a la signification déjà indiquée, correspondant à un produit de formule I' dans laquelle $R'_2$ a la signification de $R'''_2$, $R_1$ représente un atome d'hydrogène et R représente un radical benzyle produit de formule $I'_B$ que, si désiré, dans le cas ou $R'''_2$ représente un radical hydroxyalcoyle, l'on traite par un agent d'halogénation pour obtenir un produit de formule $I''_B$:

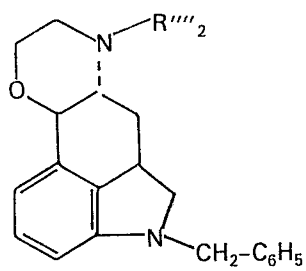

$I''_B$

dans laquelle $R''''_2$ représente un radical halogénoalcoyle, correspondant à un produit de formule I' dans laquelle $R'_2$ a la signification de $R''''_2$, $R_1$ représente un atome d'hydrogène et R représente un radical benzyle;

b) soit, dans le cas où $R''_2$ représente un atome d'hydrogène, l'on fait agir avec un agent de méthylation pour obtenir un produit de formule $I'_C$:

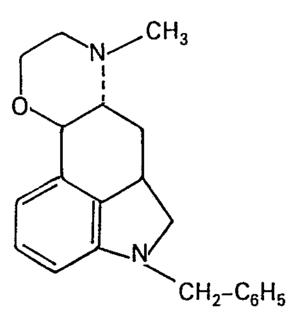

$I'_C$

correspondant à un produit de formule I′ dans laquelle $R'_2$ représente un radical méthyle, $R_1$ représente un atome d'hydrogène et R représente un radical benzyle produits de formule $I'_A$, $I'_B$, $I''_B$ et $I'_C$ que, si désiré, l'on hydrogènolyse pour obtenir un produit de formule $I'_D$:

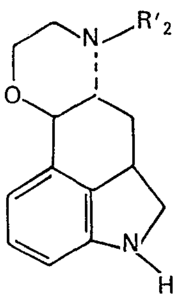

$I'_D$

dans laquelle $R'_2$ a la signification déjà indiquée, correspondant à la signification du substituant de l'atome d'azote dans les produits de formule $I'_A$, $I'_B$, $I''_B$ et $I'_C$, produit de formule $I'_D$ correspondant à un produit de formule I′ dans laquelle $R'_2$ à la valeur indiquée ci-dessus et R et $R_1$ représentent un atome d'hydrogène, produit de formule $I'_D$ que, si désiré,
- soit l'on isole,
- soit l'on fait réagir avec un halogénure de formule VII:

Hal-R′ VII

dans laquelle Hal représente un atome de chlore, de brome ou d'iode et R′ représente un radical alcoyle renfermant de 1 à 5 atomes de carbone ou un radical aralcoyle renfermant de 7 à 12 atomes de carbone, éventuellement substituée, pour obtenir un produit de formule $I''_D$:

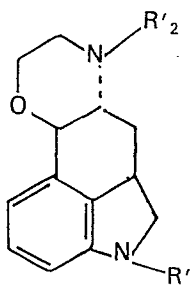

$I''_D$

dans laquelle R′ et $R'_2$ ont la signification déjà indiquée, correspondant à un produit de formule I′ dans laquelle $R'_2$ a la valeur indiquée ci-dessus, $R_1$ représente un atome d'hydrogène et R a la valeur de R′, puis, si désiré, salifie les produits de formule $I'_A$, $I'_B$, $I''_B$, $I'_C$, $I'_D$ $I''_D$.

7. Procédé selon la revendication 6, caractérisé en ce que:
a) la cyclisation du produit de formule IV est réalisée à l'aide d'hydrure de sodium,
b) l'on réduit le produit de formule V à l'aide d'hydrure d'aluminium-lithium,
c) l'hydrogénolyse des produits de formule $I'_A$, $I'_B$, $I''_B$, et $I'_C$ est réalisée à l'aide d'hydrogène en présence de palladium.

8. Médicaments, caractérisés en ce qu'ils sont constitués par les dérivés de l'indolobenzoxazine, tels que définis par la formule I′ de la revendication 1, ainsi que par leurs sels d'addition avec les acides pharmaceutiquement acceptables.

9. Médicaments, caractérisés en ce qu'ils sont constitués par les dérivés de l'indolobenzoxazine, tels que définis à la revendication 2 ou 3, ainsi que par leurs sels d'addition avec les acides pharmaceutiquement acceptables.

10. Médicaments, caractérisés en ce qu'ils sont constitués par les dérivés de l'indolobenzoxazine, tels que définis à la revendication 4, ainsi que par leurs sels d'addition avec les acides pharmaceutiquement acceptables.

11. Médicaments, caractérisés en ce qu'ils sont constitués par les dérivés de l'indolobenzoxazine, tels que définis dans la revendication 5, pharmaceutiquement acceptables.

12. Compositions pharmaceutiques, caractérisées en ce qu'elles renferment, à titre de principe actif, l'un au moins des médicaments, tels que définis à l'une des revendications 8, 9, 10 ou 11.

**Revendications**
Pour l'Etat contractant AT

1. Procédé pour préparer des dérivés de l'indolobenzoxazine, ainsi que leurs sels d'addition avec les acides minéraux ou organiques répondant à la formule générale I′:

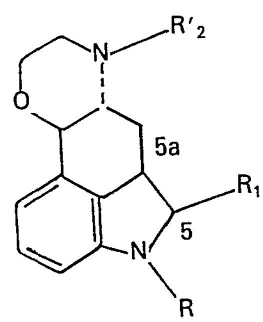

I′

dans laquelle R représente un atome d'hydrogène, un radical alcoyle renfermant de 1 à 5 atomes de carbone, ou un radical aralcoyle renfermant de 7 à 12 atomes de carbone, éventuellement substitué par des radicaux chloro, bromo, méthyl, éthyl, méthoxy, trifluorométhyl ou méthylthio, $R_1$ représente un atome d'hydrogène, de chlore ou de brome, $R'_2$ représente un atome d'hydrogène, un radical alcoyle renfermant de 1 à 5 atomes de carbone, éventuellement substitué par un atome d'halogène ou par un radical hydroxy, $R'_2$ représente encore un radical cycloalcoylalcoyle renfermant de 4 à 7 atomes de carbone, alcényle ou alcynyle renfermant de 3 à 7 atomes de carbone, ou aralcoyle renfermant de 7 à 12 atomes de carbone, éventuellement substitué

par des radicaux chloro, bromo, méthyl, éthyl, méthoxy, trifluorométhyl ou méthylthio, caractérisé en ce que l'on réduit un produit de formule II:

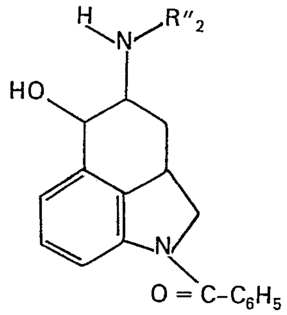

II

dans laquelle $R''_2$ représente un atome d'hydrogène, un radical alcoyle renfermant de 1 à 5 atomes de carbone, cycloalcoylalcoyle renfermant de 4 à 7 atomes de carbone, ou aralcoyle renfermant de 7 à 12 atomes de carbone, éventuellement substitué pour obtenir un produit de formule III:

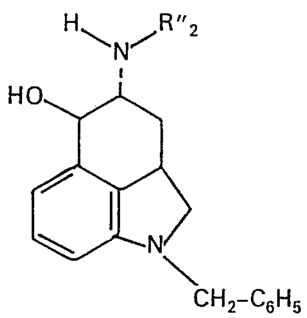

III

dans laquelle $R''_2$ a la signification déjà indiquée, que l'on fait réagir avec le chlorure de chloroacétyle pour obtenir un produit de formule IV:

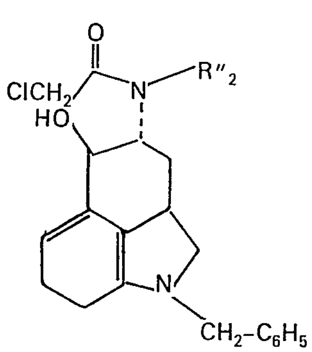

IV

dans laquelle $R''_2$ a la signification déjà indiquée, que l'on cyclise pour obtenir un produit de formule V:

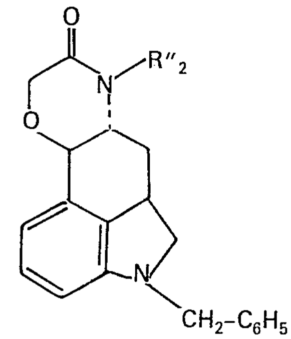

V

dans laquelle $R''_2$ a la signification déjà indiquée, que l'on réduit pour obtenir un produit de formule $I'_A$:

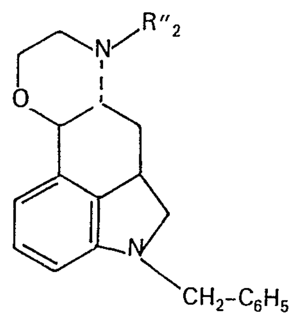

$I'_A$

dans laquelle $R''_2$ a la signification déjà indiquée, correspondant à un produit de formule I', dans laquelle $R'_2$ à la signification de $R''_2$, $R_1$ représente un atome d'hydrogène et R représente un radical benzyle, produit de formule $I'_A$ que, le cas échéant,

a) soit dans le cas, où $R''_2$ représente un atome d'hydrogène, l'on fait réagir avec un halogénure de formule VI:

$Hal\text{-}R'''_2$ VI

dans laquelle Hal représente un atome de chlore, de brome ou d'iode, et $R'''_2$ a la signification de $R'_2$ déjà indiquée à l'exception de l'hydrogène et d'halogénoalcoyle, pour obtenir un produit de formule $I'_B$:

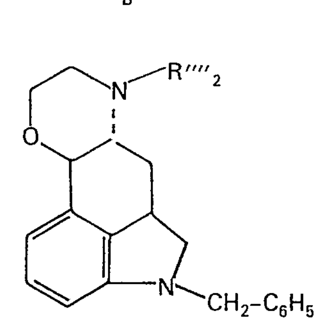

$I''_B$

dans laquelle $R'''_2$ a la signification déjà indiquée, correspondant à un produit de formule I' dans laquelle $R'_2$ a la signification de $R'''_2$, $R_1$ représente un atome d'hydrogène et R représente un radical

benzyle, produit de formule $I'_B$ que, si désiré, dans le cas ou $R'''_2$ représente un radical hydroxyalcoyle, l'on traite par un agent d'halogénation pour obtenir un produit de formule $I''_B$:

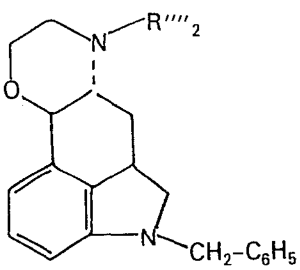

$I''_B$

dans laquelle $R''''_2$ représente un radical halogénoalcoyle, correspondant à un produit de formule I' dans laquelle $R'_2$ à la signification de $R''''_2$, R' représente un atome d'hydrogène et R représente un radical benzyle;

b) soit, dans le cas où $R''_2$ représente un atome d'hydrogène, l'on fait agir avec un agent de méthylation, pour obtenir un produit de formule $I'_C$:

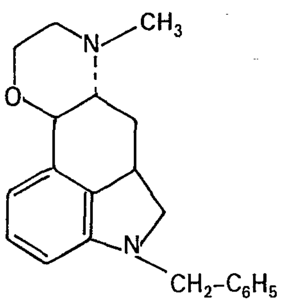

$I'_C$

correspondant à un produit de formule I' dans laquelle $R'_2$ représente un radical méthyle, $R_1$ représente un atome d'hydrogène et R représente un radical benzyle, produits de formule $I'_A$, $I'_B$, $I''_B$ et $I'_C$ que, si désiré, l'on hydrogènolyse, pour obtenir un produit de formule $I'_D$:

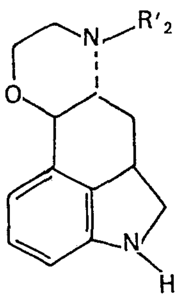

$I'_D$

dans laquelle $R'_2$ a la signification déjà indiquée, correspondant à la signification du substituant de l'atome d'azote dans les produits de formule $I'_A$, $I'_B$, $I''_B$ et $I'_C$, produit de formule $I'_D$ correspondant à un produit de formule I' dans laquelle $R'_2$ à la valeur indiquée ci-dessus et R et $R_1$ représentent un atome d'hydrogène produit de formule $I'_D$ que, si désiré,

- soit l'on isole,
- soit l'on fait réagir avec un halogénure de formule VII:

Hal–R' VII

dans laquelle Hal représente un atome de chlore, de brome ou d'iode et R' représente un radical alcoyle renfermant de 1 à 5 atomes de carbone ou un radical aralcoyle renfermant de 7 à 12 atomes de carbone, éventuellement substitué, pour obtenir un produit de formule $I''_D$:

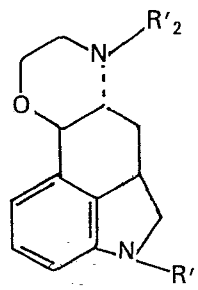

$I''_D$

dans laquelle R' et $R'_2$ ont la signification déjà indiquée, correspondant à un produit de formule I' dans laquelle $R'_2$ a la valeur indiquée ci-dessus, $R'_1$ représente un atome d'hydrogène et R a la valeur de $R_1$, puis, si désiré, salifie les produits de formule $I'_A$, $I'_B$, $I''_B$, $I'_C$, $I'_D$ ou $I''_D$.

2. Procédé selon la revendication 1, pour préparer les dérivés de l'indolobenzoxazine de formule I' ainsi que leurs sels répondant à la formule générale I:

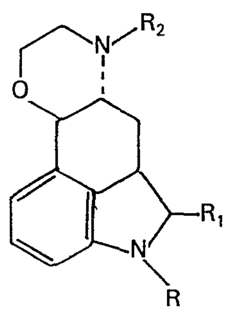

I

dans laquelle R et $R_1$ sont définis comme à la revendication 1 et $R_2$ représente un atome d'hydrogène, un radical alcoyle renfermant de 1 à 5 atomes de carbone, cycloalcoylalcoyle renfermant de 4 à 7 atomes de carbone, alcényle ou alcynyle renfermant de 3 à 7 atomes de carbone, ou aralcoyle renfermant de 7 à 12 atomes de carbone, éventuellement substitué par des radicanx chloro, bromo, méthyl, éthyl, méthoxy, trifluorométhyl ou méthylthio, caractérisé en ce que l'on réduit un produit de formule II:

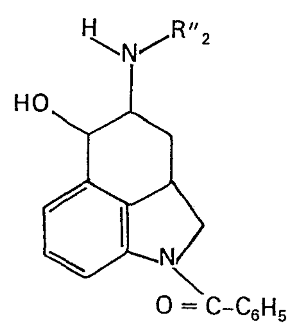

II

dans laquelle $R''_2$ représente un atome d'hydrogène, un radical alcoyle renfermant de 1 à 5 atomes de carbone, cycloalcoylalcoyle renfermant de 4 à 7 atomes de carbone, ou aralcoyle renfermant de 7 à 12 atomes de carbone, éventuellement substitué, pour obtenir un produit de formule III:

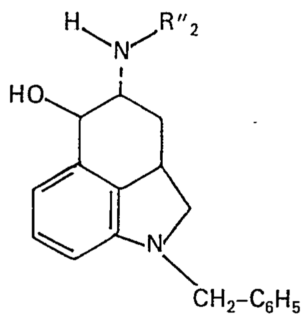

III

dans laquelle $R''_2$ a la signification déjà indiquée, que l'on fait réagir avec le chlorure de chloroacétyle pour obtenir un produit de formule IV:

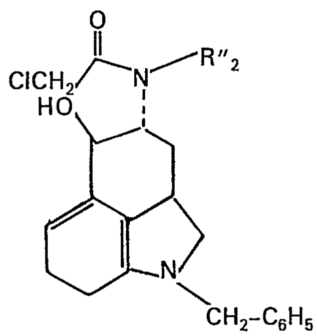

IV

dans laquelle $R''_2$ a la signification déjà indiquée, que l'on cyclise pour obtenir un produit de formule V:

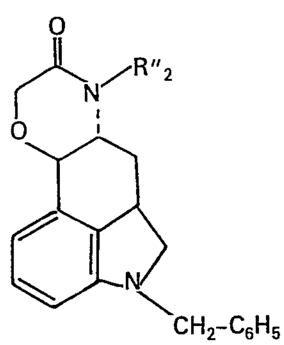

V

dans laquelle $R''_2$ a la signification déjà indiquée, que l'on réduit pour obtenir un produit de formule $I'_A$:

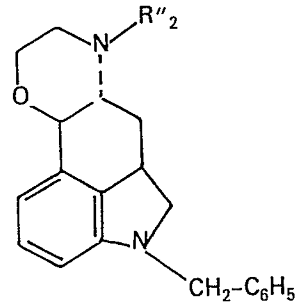

$I'_A$

dans laquelle $R''_2$ a la signification déjà indiquée, correspondant à un produit de formule I', dans laquelle $R'_2$ à la signification de $R''_2$, $R_1$ représente un atome d'hydrogène et R représente un radical benzyle, produit de formule $I'_A$ que, le cas échéant,

a) soit dans le cas où $R''_2$ représente un atome d'hydrogène, l'on fait réagir avec un halogénure de formule VI:

$Hal-R'_2$ VI

dans laquelle Hal représente un atome de chlore, de brome ou d'iode, et $R'_2$ a la signification de $R_2$ déjà indiquée à l'exception de l'hydrogène, pour obtenir un produit de formule $I_B$:

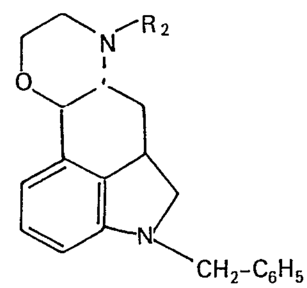

$I_B$

dans laquelle $R'_2$ a la signification déjà indiquée, correspondant à un produit de formule I dans laquelle $R_2$ a la signification de $R'_2$, $R_1$ représente un atome d'hydrogène et R représente un radical benzyle,

b) soit, dans le cas où $R''_2$ représente un atome d'hydrogène, l'on fait agir avec un agent de méthylation, pour obtenir un produit de formule $I'_C$:

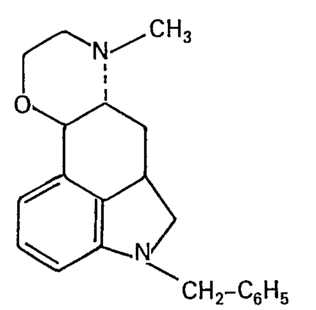

$I'_C$

correspondant à un produit de formule I dans laquelle $R_2$ représente un radical méthyle, $R_1$ représente un atome d'hydrogène et R représente un radical benzyle produits de formule $I'_A$, $I_B$ et $I'_C$ que, si désiré, l'on hydrogènolyse, pour obtenir un produit de formule $I_D$:

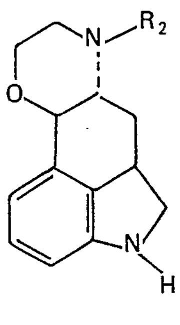

$I'_D$

dans laquelle $R_2$ a la signification déjà indiquée, correspondant à la signification du substituant de l'atome d'azote dans les produits de formule $I'_A$, $I_B$ et $I'_C$ produit de formule $I_D$ correspondant à un produit de formule I dans laquelle $R_2$ à la valeur indiquée ci-dessus, R et $R_1$ représentent un atome d'hydrogène, puis, si désiré, salifie les produits de formule $I'_A$, $I'_B$, $I'_C$ ou $I_D$.

3. Procédé selon la revendication 2, caractérisé en ce que:
a) la cyclisation du produit de formule IV est réalisé à l'aide d'hydrure de sodium,
b) l'on réduit le produit de formule V à l'aide d'hydrure d'aluminium-lithium,
c) l'hydrogénolyse des produits de formule $I'_A$, $I_B$ et $I'_C$ est réalisée à l'aide d'hydrogène en présence de palladium.

4. Procédé selon la revendication 2, caractérisé en ce que l'on utilise au départ un produit de formule II dans laquelle $R''_2$ représente un atome d'hydrogène ou un radical alcoyle renfermant de 1 à 5 atomes de carbone ou cycloalcoylalcoyle renfermant de 4 à 7 atomes de carbone ou un réactif de formule VI dans laquelle $R'_2$ représente un radical alcoyle renfermant de 1 à 5 atomes de carbone ou cycloalcoylalcoyle renfermant de 4 à 7 atomes de carbone.

5. Procédé selon la revendication 2, caractérisé en ce que l'on utilise au départ un produit de formule II dans laquelle $R''_2$ représente un atome d'hydrogène ou un radical alcoyle renfermant de 1 à 5 atomes de carbone ou un réactif de formule VI dans laquelle $R'_2$ représente un radical alcoyle renfermant de 1 à 5 atomes de carbone.

6. Procédé selon l'une quelconque des revendications 1 à 5, caractérisé en ce que l'on prépare la 5, 5a, 6, 6a (RS), 7, 8, 9, 10a (RS)-octahydro 7-propyl 4H-indolo [3,4-g, h] [1,4]benzoxazine ainsi que ses sels d'addition avec les acides minéraux ou organiques.

**Patentansprüche**

Für die Vertragsstaaten BE, CH, DE, FR, GB, IT, LI, NL, SE

1. Indolobenzoxazin-Derivate sowie deren Additionssalze mit Mineral- oder organischen Säuren, dadurch gekennzeichnet, daß sie der allgemeinen Formel I' entsprechen

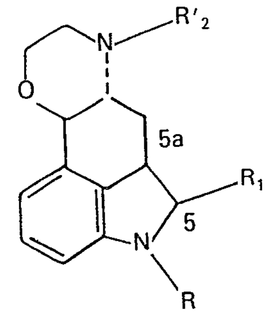

(I')

worin R ein Wasserstoffatom, einen Alkylrest mit 1 bis 5 Kohlenstoffatomen oder einen Aralkylrest mit 7 bis 12 Kohlenstoffatomen, gegebenenfalls substituiert durch Chlor-, Brom-, Methyl-, Ethyl-, Methoxy-, Trifluormethyl- oder Methylthioreste, bedeutet; $R_1$ ein Wasserstoffatom-, Chlor- oder Bromatom darstellt; $R'_2$ ein Wasserstoffatom, einen Alkylrest mit 1 bis 5 Kohlenstoffatomen, der gegebenenfalls durch ein Halogenatom oder durch einen Hydroxyrest substituiert ist, bedeutet; $R'_2$ auch einen Cycloalkalkylrest mit 4 bis 7 Kohlenstoffatomen, einen Alkenyl- oder Alkinylrest mit 3 bis 7 Kohlenstoffatomen oder einen Aralkylrest mit 7 bis 12 Kohlenstoffatomen, gegebenenfalls substituiert durch Chlor-, Brom-, Methyl-, Ethyl-, Methoxy-, Trifluormethyl- oder Methylthioreste, bedeutet.

2. Indolobenzoxazin-Derivate gemäß Anspruch 1 sowie deren Additionssalze mit Mineral- oder organischen Säuren, dadurch gekennzeichnet, daß sie der allgemeinen Formel I

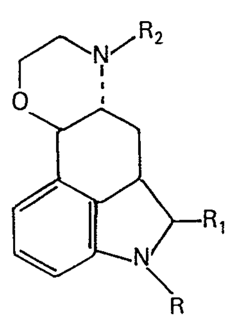

(I)

entsprechen, worin R und $R_1$ wie in Anspruch 1 definiert sind und $R_2$ ein Wasserstoffatom, einen Alkylrest mit 1 bis 5 Kohlenstoffatomen, einen Cycloalkalkylrest mit 4 bis 7 Kohlenstoffatomen, einen Alkenyl- oder Alkinylrest mit 3 bis 7 Kohlenstoffatomen oder einen Aralkylrest mit 7 bis 12 Kohlenstoffatomen, gegebenenfalls substituiert durch Chlor-, Brom-, Methyl-, Ethyl-, Methoxy-, Trifluormethyl- oder Methylthioreste, bedeutet.

3. Indolobenzoxazin-Derivate gemäß Anspruch 2 sowie deren Additionssalze mit Mineral- oder organischen Säuren, dadurch gekennzeichnet, daß R ein Wasserstoffatom bedeutet und $R_2$ ein Wasserstoffatom oder einen Alkylrest mit 1 bis 5 Kohlenstoffatomen oder einen Cycloalkalkylrest mit 4 bis 7 Kohlenstoffatomen bedeutet.

4. Indolobenzoxazin-Derivate gemäß Anspruch 2 sowie deren Additionssalze mit Mineral- oder organischen Säuren, dadurch gekennzeichnet, daß R ein Wasserstoffatom bedeutet, $R_1$ ein Wasserstoffatom oder Bromatom darstellt und $R_2$ ein Wasserstoffatom oder einen Alkylrest mit 1 bis 5 Kohlenstoffatomen bedeutet.

5. 5,5a,6,6a(RS),7,8,9,10a(RS)-Octahydro-7-propyl-4H-indolo[3.4-g.h][1.4]benzoxazin sowie dessen Additionssalze mit Mineral- oder organischen Säuren.

6. Verfahren zur Herstellung der Derivate der Formel I′ gemäss Anspruch 1 sowie von deren Salzen, dadurch gekennzeichnet, dass man ein Produkt der Formel II worin $R''_2$ ein Wasserstoffatom,

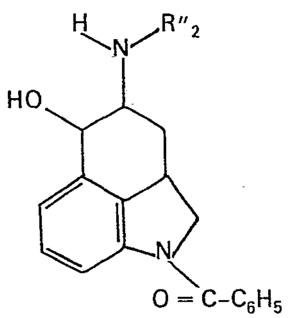

(II)

tom, einen Alkylrest mit 1 bis 5 Kohlenstoffatomen, einen Cycloalkalkylrest mit 4 bis 7 Kohlenstoffatomen oder einen Aralkylrest mit 7 bis 12 Kohlenstoffatomen, der gegebenenfalls substituiert ist, bedeutet, reduziert, um ein Produkt der Formel III

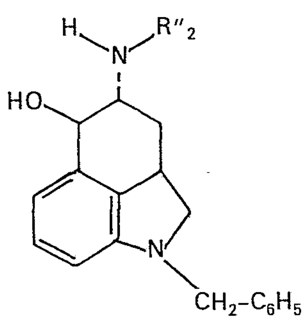

(III)

zu erhalten, worin $R''_2$ die angegebene Bedeutung besitzt, das man mit Chloracetylchlorid umsetzt, um ein Produkt der Formel IV

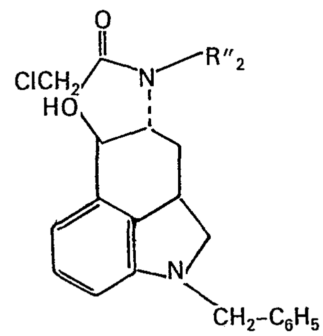

(IV)

zu erhalten, worin $R''_2$ die angegebene Bedeutung besitzt, das man cyclisiert, um ein Produkt der Formel V

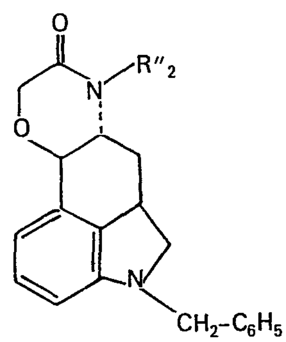

(V)

zu erhalten, worin $R''_2$ die angegebene Bedeutung besitzt, das man reduziert, um ein Produkt der Formel $I'_A$

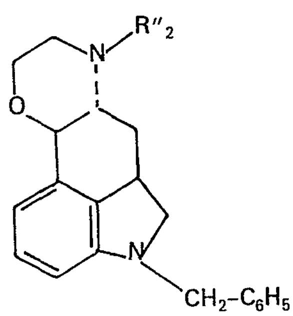

( $I'_A$ )

zu erhalten, worin $R''_2$ die angegebene Bedeutung besitzt, entsprechend einem Produkt der Formel I′, worin $R'_2$ die Bedeutung von $R''_2$ besitzt, $R_1$ ein Wasserstoffatom bedeutet und R einen Benzylrest darstellt, welches Produkt der Formel $I'_A$ man gegebenenfalls

(a) entweder, wenn $R''_2$ ein Wasserstoffatom bedeutet, mit einem Halogenid der Formel VI

$$Hal\text{-}R'''_2 \qquad (VI)$$

umsetzt, worin Hal ein Chlor-, Brom- oder jodatom darstellt, und $R'''_2$ die angegebene Bedeutung von $R'_2$ mit Ausnahme von Wasserstoff und Halogenalkyl besitzt, um ein Produkt der Formel $I'_B$

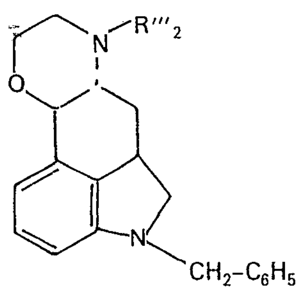

($I'_B$)

zu erhalten, worin $R'''_2$ die angegebene Bedeutung besitzt, entsprechend einem Produkt der Formel I', worin $R'_2$ die Bedeutung von $R'''_2$ hat, $R_1$ ein Wasserstoffatom bedeutet und R für einen Benzylrest steht, welches Produkt der Formel $I'_B$ man gegebenenfalls, wenn $R'''_2$ einen Hydroxyalkylrest bedeutet, mit einem Halogenierungsmittel behandelt, um ein Produkt der Formel $I''_B$

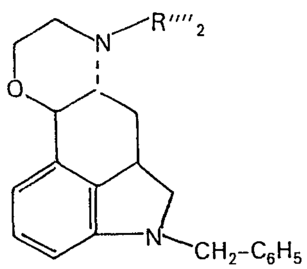

($I''_B$)

zu erhalten, worin $R''''_2$ einen Halogenalkylrest bedeutet, entsprechend einem Produkt der Formel I', worin $R'_2$ die Bedeutung von $R''''_2$ besitzt, $R_1$ ein Wasserstoffatom bedeutet und R für einen Benzylrest steht;

(b) oder, wenn $R''_2$ ein Wasserstoffatom bedeutet, mit einem Methylierungsmittel umsetzt, um ein Produkt der Formel $I'_C$

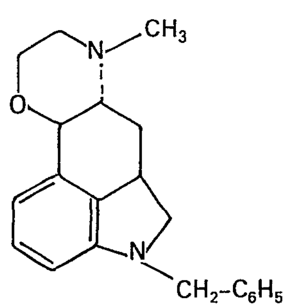

($I'_C$)

zu erhalten, entsprechend einem Produkt der Formel I', worin $R'_2$ einen Methylrest darstellt, $R_1$ ein Wasserstoffatom bedeutet und R für einen Benzylrest steht, welche Produkte der Formel $I'_A$, $I'_B$, $I''_B$ und $I'_C$ man gewünschtenfalls einer Hydrogenolyse unterzieht, um ein Produkt der Formel $I'_D$

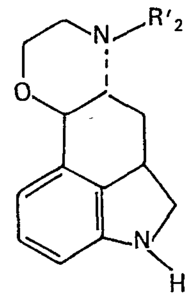

($I'_D$)

zu erhalten, worin $R'_2$ die angegebene Bedeutung, entsprechend der Bedeutung des Substituenten des Stickstoffatoms in den Produkten der Formel $I'_A$, $I'_B$, $I''_B$ und $I'_C$ besitzt, wobei das Produkt der Formel $I'_D$ einem Produkt der Formel I' entspricht, worin $R'_2$ die angegebene Bedeutung besitzt und R und $R_1$ ein Wasserstoffatom bedeuten, welches Produkt der Formel $I'_D$ man gewünschtenfalls
entweder isoliert
oder mit einem Halogenid der Formel VII

Hal-R' (VII)

umsetzt, worin Hal ein Chlor-, Brom- oder Jodatom bedeutet und R' einen Alkylrest mit 1 bis 5 Kohlenstoffatomen oder einen Aralkylrest mit 7 bis 12 Kohlenstoffatomen, der gegebenenfalls substituiert ist, bedeutet, um ein Produkt der Formel $I''_D$

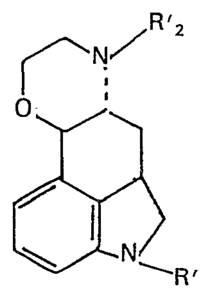

($I''_D$)

zu erhalten, worin R' und $R'_2$ die angegebene Bedeutung besitzen, entsprechend einem Produkt der Formel I', worin $R'_2$ die angegebene Bedeutung besitzt, $R_1$ ein Wasserstoffatom bedeutet und R die Bedeutung von R' besitzt, und danach gewünschtenfalls die Produkte der Formeln $I'_A$, $I'_B$, $I''_B$, $I'_C$, $I'_D$ und $I''_D$ in ein Salz überführt.

7. Verfahren gemäß Anspruch 6, dadurch gekennzeichnet, daß
(a) die Cyclisierung des Produktes der Formel IV mit Natriumhydrid durchgeführt wird,
(b) man das Produkt der Formel V mit Aluminiumlithiumhydrid reduziert;
(c) die Hydrogenolyse der Produkte der Formeln $I'_A$, $I'_B$, $I''_B$ und $I'_C$ mit Wasserstoff in Gegenwart von Palladium durchgeführt wird.

8. Arzneimittel, dadurch gekennzeichnet, daß sie aus den neuen Indolobenzoxazin-Derivaten der Formel I' gemäß Anspruch 1 sowie deren Additionssalzen mit pharmazeutisch verträglichen Säuren bestehen.

9. Arzneimittel, dadurch gekennzeichnet, daß sie aus den Indolobenzoxazin-Derivaten gemäß

Anspruch 2 oder 3 sowie aus deren Additionssalzen mit pharmazeutisch verträglichen Säuren bestehen.

10. Arzneimittel, dadurch gekennzeichnet, daß sie aus den Indolobenzoxaxin-Derivaten gemäß Anspruch 4 sowie aus deren Additionssalzen mit pharmazeutisch verträglichen Säuren bestehen.

11. Arzneimittel, dadurch gekennzeichnet, daß sie aus den pharmazeutisch verträglichen Indolobenzoxazin-Derivaten gemäß Anspruch 5 bestehen.

12. Pharmazeutische Zusammensetzungen, dadurch gekennzeichnet, daß sie als Wirkstoff zumindest eines der Arzneimittel gemäß einem der Ansprüche 8, 9, 10 oder 11 enthalten.

**Patentansprüche**
Für den Vertragsstaat: AT

1. Verfahren zur Herstellung von Indolobenzoaxin-Derivaten sowie von deren Additionssalzen mit Mineral- oder organischen Säuren der allgemeinen Formel I'

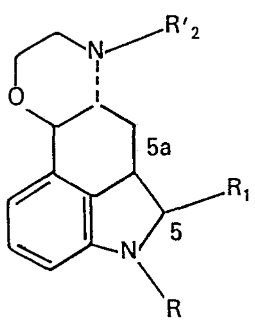

(I')

worin R ein Wasserstoffatom, einen Alkylrest mit 1 bis 5 Kohlenstoffatomen oder einen Aralkylrest mit 7 bis 12 Kohlenstoffatomen, gegebenenfalls substituiert durch Chlor-, Brom-, Methyl-, Ethyl-, Methoxy-, Trifluormethyl- oder Methylthioreste, bedeutet, $R_1$ ein Wasserstoff-, Chlor- oder Bromatom darstellt, $R'_2$ ein Wasserstoffatom, einen Alkylrest mit 1 bis 5 Kohlenstoffatomen, der gegebenenfalls substituiert ist durch ein Halogenatom oder einen Hydroxyrest, bedeutet, $R'_2$ auch einen Cycloalkalkylrest mit 4 bis 7 Kohlenstoffatomen, einen Alkenyl- oder Alkinylrest mit 3 bis 7 Kohlenstoffatomen oder einen Aralkylrest mit 7 bis 12 Kohlenstoffatomen, gegebenenfalls substituiert durch Chlor-, Brom-, Methyl-, Ethyl-, Methoxy-, Trifluormethyl- oder Methylthioreste, bedeutet, dadurch gekennzeichnet, daß man ein Produkt der Formel II

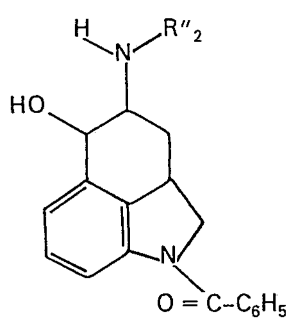

(II)

worin $R''_2$ ein Wasserstoffatom, einen Alkylrest mit 1 bis 5 Kohlenstoffatomen, einen Cycloalkalkylrest mit 4 bis 7 Kohlenstoffatomen oder einen Aralkylrest mit 7 bis 12 Kohlenstoffatomen, der gegebenenfalls substituiert ist, bedeutet, reduziert, um ein Produkt der Formel III

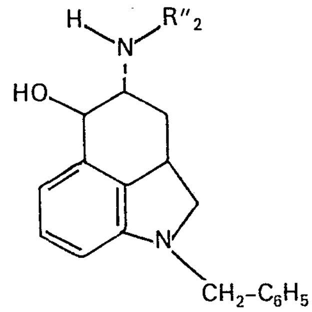

(III)

zu erhalten, worin $R''_2$ die angegebene Bedeutung besitzt, das man mit Chloracetylchlorid umsetzt, um ein Produkt der Formel IV

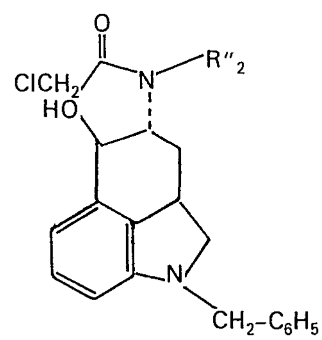

(IV)

zu erhalten, worin $R''_2$ die angegebene Bedeutung besitzt, das man cyclisiert, um ein Produkt der Formel V

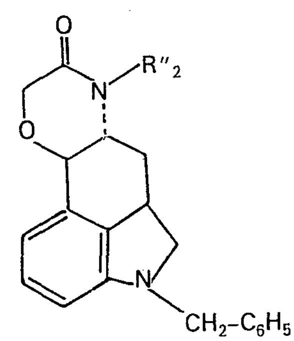

(V)

zu erhalten, worin $R''_2$ die angegebene Bedeutung besitzt, das man reduziert, um ein Produkt der Formel $I'_A$

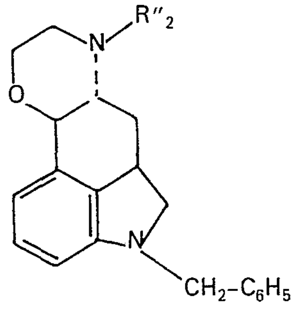

($I'_A$)

zu erhalten, worin $R''_2$ die angegebene Bedeutung besitzt, entsprechend einem Produkt der Formel I', worin $R'_2$ die Bedeutung von $R''_2$ besitzt, $R_1$ ein Wasserstoffatom darstellt und R für einen Benzylrest steht, welches Produkt der Formel $I'_A$ man gegebenenfalls

a) entweder, wenn $R''_2$ ein Wasserstoffatom bedeutet, mit einem Halogenid der Formel VI

$$\text{Hal-}R'''_2 \qquad (VI)$$

umsetzt, worin Hal ein Chlor-, Brom- oder Jodatom bedeutet und $R'''_2$ die angegebene Bedeutung von $R'_2$ mit Ausnahme von Wasserstoff und Halogenalkyl besitzt, um ein Produkt der Formel $I'_B$

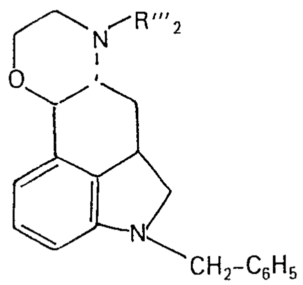

$(I'_B)$

zu erhalten, worin $R'''_2$ die angegebene Bedeutung besitzt, entsprechend einem Produkt der Formel (I'), worin $R'_2$ die Bedeutung von $R'''_2$ hat, $R_1$ ein Wasserstoffatom darstellt und R für einen Benzylrest steht, welches Produkt der Formel $I'_B$ man gewünschtenfalls, wenn $R'''_2$ einen Hydroxyalkylrest bedeutet, mit einem Halogenierungsmittel behandelt, um ein Produkt der Formel $I''_B$

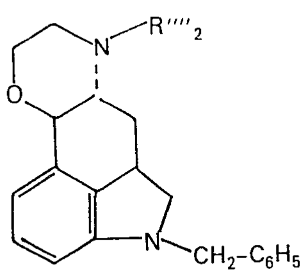

$(I''_B)$

zu erhalten, worin $R''''_2$ einen Halogenalkylrest darstellt, entsprechend einem Produkt der Formel I', worin $R'_2$ die Bedeutung von $R''''_2$ besitzt, $R_1$ ein Wasserstoffatom bedeutet und R für einen Benzylrest steht;

b) oder, wenn $R''_2$ ein Wasserstoffatom bedeutet, mit einem Methylierungsmittel umsetzt, um ein Produkt der Formel $I'_C$

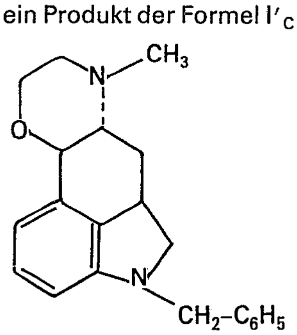

$(I'_C)$

zu erhalten, entsprechend einem Produkt der Formel I', worin $R'_2$ einen Methylrest bedeutet, $R_1$ ein Wasserstoffatom darstellt und R für einen Benzylrest steht, welche Produkte der Formeln $I'_A$, $I'_B$, $I''_B$ und $I'_C$ man gewünschtenfalls einer Hydrogenolyse unterzieht, um ein Produkt der Formel $I'_D$

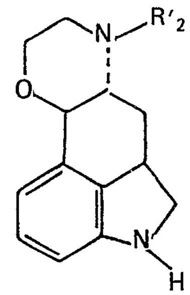

$(I'_D)$

zu erhalten, worin $R'_2$ die angegebene Bedeutung entsprechend der Bedeutung des Substituenten des Stickstoffatoms in den Produkten der Formeln $I'_A$, $I'_B$, $I''_B$ und $I'_C$ besitzt, wobei das Produkt der Formel $I'_D$ einem Produkt der Formel I' entspricht, worin $R'_2$ die vorstehend angegebene Bedeutung hat und R und $R_1$ ein Wasserstoffatom darstellen,

welches Produkt der Formel $I'_D$ man gewünschtenfalls

entweder isoliert

oder mit einem Halogenid der Formel VII

$$\text{Hal-}R' \qquad (VII)$$

umsetzt, worin Hal ein Chlor-, Brom- oder Jodatom bedeutet und R' einen Alkylrest mit 1 bis 5 Kohlenstoffatomen oder einen Aralkylrest mit 7 bis 12 Kohlenstoffatomen, der gegebenenfalls substituiert ist, darstellt, um ein Produkt der Formel $I''_D$

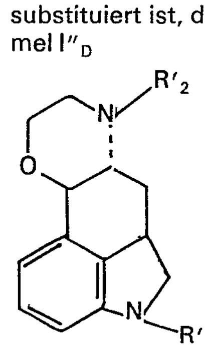

$(I''_D)$

zu erhalten, worin R' und $R'_2$ die angegebene Bedeutung besitzen, entsprechend einem Produkt der Formel I', worin $R'_2$ die vorstehend angegebene Bedeutung besitzt, $R_1$ ein Wasserstoffatom bedeutet und R die Bedeutung von R' besitzt, und danach gewünschtenfalls die Produkte der Formeln $I'_A$, $I'_B$, $I''_B$, $I'_C$, $I'_D$ oder $I''_D$ in ein Salz überführt.

2. Verfahren gemäß Anspruch 1 zur Herstellung der Indolobenzoxazin-Derivate der Formel (I') sowie von deren Salzen der allgemeinen Formel I

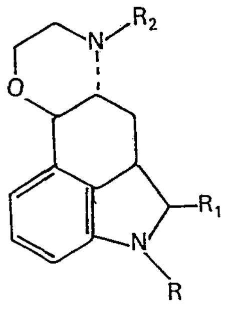

(I)

worin R und $R_1$ wie in Anspruch 1 definiert sind und $R_2$ ein Wasserstoffatom, einen Alkylrest mit 1 bis 5 Kohlenstoffatomen, einen Cycloalkalkylrest mit 4 bis 7 Kohlenstoffatomen, einen Alkenyl- oder Alkinylrest mit 3 bis 7 Kohlenstoffatomen oder einen Aralkylrest mit 7 bis 12 Kohlenstoffatomen, gegebenenfalls substituiert durch Chlor-, Brom-, Methyl-, Ethyl-, Methoxy-, Trifluormethyl- oder Methylthioreste, bedeutet, dadurch gekennzeichnet, daß man ein Produkt der Formel II

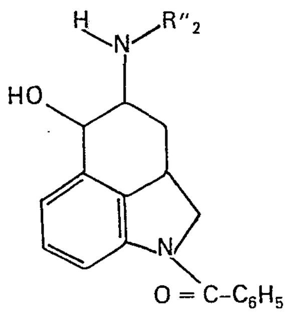

(II)

worin $R''_2$ ein Wasserstoffatom, einen Alkylrest mit 1 bis 5 Kohlenstoffatomen, einen Cycloalkalkylrest mit 4 bis 7 Kohlenstoffatomen oder einen Aralkylrest mit 7 bis 12 Kohlenstoffatomen, der gegebenenfalls substituiert ist, bedeutet, reduziert, um ein Produkt der Formel III

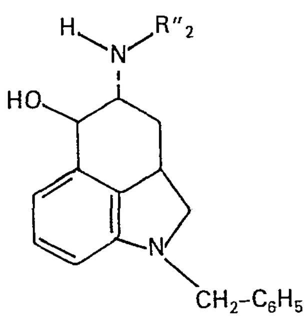

(III)

worin $R''_2$ die angegebene Bedeutung besitzt, zu erhalten, das man mit Chloracetylchlorid umsetzt, um ein Produkt der Formel IV

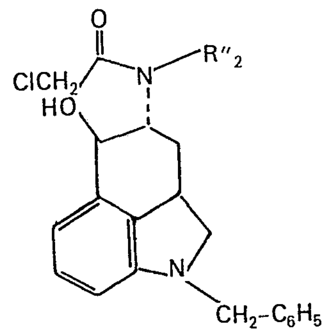

(IV)

zu erhalten, worin $R''_2$ die angegebene Bedeutung besitzt, das man cyclisiert, um ein Produkt der Formel (V)

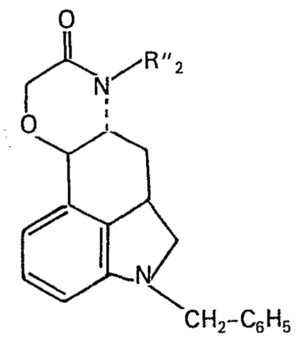

(V)

zu erhalten, worin $R''_2$ die angegebene Bedeutung besitzt, das man reduziert, um ein Produkt der Formel $I'_A$

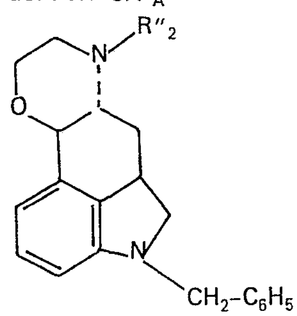

($I'_A$)

zu erhalten, worin $R''_2$ die angegebene Bedeutung besitzt, entsprechend einem Produkt der Formel I′, worin $R'_2$ die Bedeutung von $R''_2$ besitzt, $R_1$ ein Wasserstoffatom bedeutet und R für einen Benzylrest steht, welches Produkt der Formel $I'_A$ man gegebenenfalls

a) entweder, wenn $R''_2$ ein Wasserstoffatom bedeutet, mit einem Halogenid der Formel VI

Hal-$R'_2$ (VI)

worin Hal ein Chlor-, Brom- oder Jodatom bedeutet und $R'_2$ die angegebene Bedeutung von $R_2$ mit Ausnahme von Wasserstoff besitzt, umsetzt, um ein Produkt der Formel $I_B$

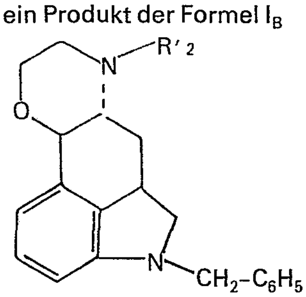

($I'_B$)

zu erhalten, worin $R'_2$ die angegebene Bedeutung hat, entsprechend einem Produkt der Formel I, worin $R_2$ die Bedeutung von $R'_2$ besitzt, $R_1$ ein Wasserstoffatom bedeutet und R für einen Benzylrest steht,

b) oder, wenn $R''_2$ ein Wasserstoffatom bedeutet, mit einem Methylierungsmittel umsetzt, um ein Produkt der Formel $I'_C$

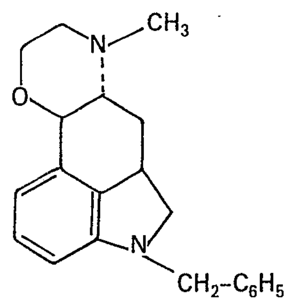

$(I'_C)$

zu erhalten, entsprechend einem Produkt der Formel I, worin $R_2$ ein Methylrest bedeutet, $R_1$ ein Wasserstoffatom darstellt und R für einen Benzylrest steht, welche Produkte der Formeln $I'_A$, $I_B$ und $I'_C$ man gewünschtenfalls einer Hydrolyse unterzieht, um ein Produkt der Formel $I_D$

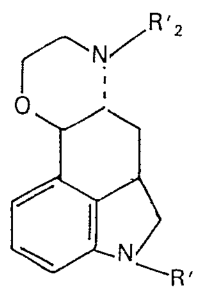

$(I_D)$

zu erhalten, worin $R_2$ die angegebene Bedeutung, entsprechend der Bedeutung des Substituenten des Stickstoffatoms in den Produkten der Formeln $I'_A$, $I_B$ und $I'_C$ besitzt, wobei das Produkt der Formel $I_D$ einem Produkt der Formel I entspricht, worin $R_2$ die vorstehend angegebene Bedeutung besitzt und R und $R_1$ ein Wasserstoffatom bedeuten, und danach gewünschtenfalls die Produkte der Formeln $I'_A$, $I_B$, $I'_C$ oder $I_D$ in ein Salz überführt.

3. Verfahren gemäß Anspruch 2, dadurch gekennzeichnet, daß

a) die Cyclisierung des Produktes der Formel IV mit Natriumhydrid durchgeführt wird,

b) man das Produkt der Formel V mit Aluminiumlithiumhydrid reduziert und

c) die Hydrogenolyse der Produkte der Formeln $I'_A$, $I_B$ und $I'_C$ mit Wasserstoff in Gegenwart von Palladium durchführt.

4. Verfahren gemäß Anspruch 2, dadurch gekennzeichnet, daß man von einem Produkt der Formel II, worin $R''_2$ ein Wasserstoffatom oder einen Alkylrest mit 1 bis 5 Kohlenstoffatomen oder einen Cycloalkalkylrest mit 4 bis 7 Kohlenstoffatomen darstellt, oder einem Reagens der Formel VI, worin $R'_2$ einen Alkylrest mit 1 bis 5 Kohlenstoffatomen oder einen Cycloalkalkylrest mit 4 bis 7 Kohlenstoffatomen bedeutet, ausgeht.

5. Verfahren gemäß Anspruch 2, dadurch gekennzeichnet, daß man von einem Produkt der Formel II, worin $R''_2$ ein Wasserstoffatom oder einen Alkylrest mit 1 bis 5 Kohlenstoffatomen bedeutet, oder einem Reagens der Formel VI, worin $R'_2$ einen Alkylrest mit 1 bis 5 Kohlenstoffatomen darstellt, ausgeht.

6. Verfahren gemäß einem der Ansprüche 1 bis 5, dadurch gekennzeichnet, daß man das 5,5a,6,6a(RS),7,8,9,10a(RS)-Octahydro-7-propyl-4H-indolo[3.4-g.h][1.4]-benzoxazin sowie dessen Additionssalze mit Mineral- oder organischen Säuren herstellt.

**Claims**

For the contracting States: BE, CH, DE, FR, GB, IT, LI, NL, S

1. Derivatives of indolobenzoxazine, as well as their salts of addition with mineral or organic acids, characterized in that they correspond to the general formula (I'):

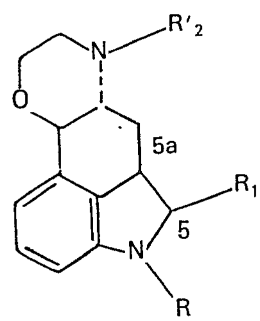

I'

in which R represents a hydrogen atom, an alkyl radical containing 1-5 carbon atoms or an aralkyl radical containing 7-12 carbon atoms, possibly substituted by chloro, bromo, methyl, ethyl, methoxy, trifluoromethyl or methylthio radicals, $R_1$ represents a hydrogen, chlorine or bromine atom, $R'_2$ represents a hydrogen atom, an alkyl radical containing 1-5 carbon atoms, possibly substituted by a halogen atom or by a hydroxy radical, $R'_2$ further represents a cycloalkylalkyl radical containing 4-7 carbon atoms, an alkenyl or alkynyl radical containing 3-7 carbon atoms, or an aralkyl radical containing 7-12 carbon atoms, possibly substituted by chloro, bromo, methyl, ethyl, methoxy, trifluoromethyl or methylthio radicals.

2. Derivatives of indolobenzoxazine according to Claim 1, as well as their salts of addition with mineral or organic acids, characterized in that they correspond to the general formula (I):

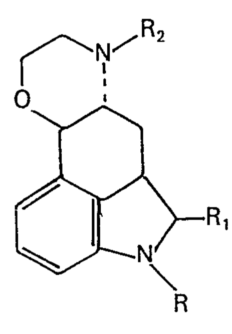

I

in which R and $R_1$ are defined as in Claim 1 and $R_2$ represents a hydrogen atom, an alkyl radical containing 1-5 carbon atoms, a cycloalkylalkyl containing 4-7 carbon atoms, an alkenyl or alkynyl radical containing 3-7 carbon atoms, or an aralkyl radical containing 7-12 carbon atoms, possibly substituted by chloro, bromo, methyl, ethyl, methoxy, trifluoromethyl or methylthio radicals.

3. Derivatives of indolobenzoxazine according to Claim 2, as well as their salts of addition with mineral or organic acids, characterized in that R represents a hydrogen atom and $R_2$ represents a hydrogen atom or an alkyl radical containing 1-5 carbon atoms or a cycloalkylalkyl radical containing 4-7 carbon atoms.

4. Derivatives of indolobenzoxazine according to Claim 2, as well as their salts of addition with mineral or organic acids, characterized in that R represents a hydrogen atom, $R_1$ represents a hydrogen atom or a bromine atom, and $R_2$ represents a hydrogen atom or an alkyl radical containing 1-5 carbon atoms.

5. 5,5a,6,6a(RS),7,8,9,10a(RS)-octahydro-7-propyl-4H-indolo-[3,4-g.h]-[1,4]benzoxazine, as well as its salts of addition with mineral or organic acids.

6. Preparation process for the derivatives, as defined by formula (I′) of Claim 1, as well as their salts, characterized in that a product with the formula (II):

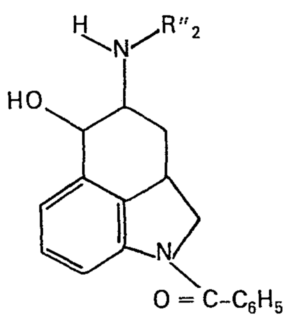

II

in which $R''_2$ represents a hydrogen atom, an alkyl radical containing 1-5 carbon atoms, a cycloalkylalkyl radical containing 4-7 carbon atoms, or an aralkyl radical containing 7-12 carbon atoms, possibly substituted, is reduced so as to obtain a product with the formula (III):

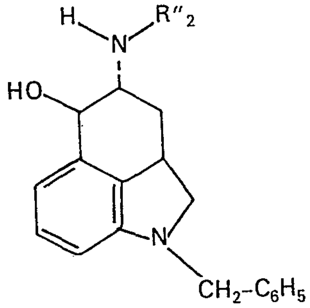

III

in which $R''_2$ has the significance already indicated, which is made to react with chloroacetyl chloride so as to obtain a product with the formula (IV):

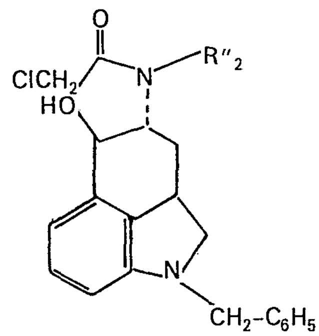

IV

in which $R''_2$ has the significance already indicated, which is cyclized so as to obtain a product with the formula (V):

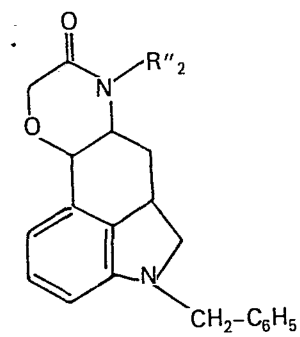

V

in which $R''_2$ has the significance already indicated, which is reduced so as to obtain a product with the formula ($I'_A$):

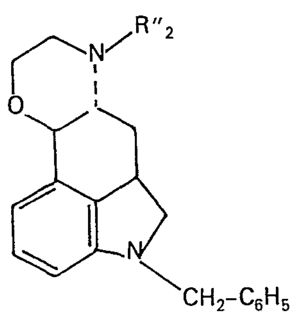

$I'_A$

in which $R''_2$ has the significance already indicated, corresponding to a product with the formula (I′), in which $R'_2$ has the significance of $R''_2$, $R_1$ represents a hydrogen atom and R represents a benzyl radical, which product with the formula ($I'_A$), if applicable,

a) either when $R''_2$ represents a hydrogen atom, is made to react with a halide with the formula (VI):

$$Hal\text{-}R'''_2 \qquad (VI)$$

in which Hal represents a chlorine, bromine or iodine atom, and R‴ has the significance of $R'_2$ already indicated, with the exception of hydrogen and an alkyl halide, so as to obtain a product with the formula ($I'_B$):

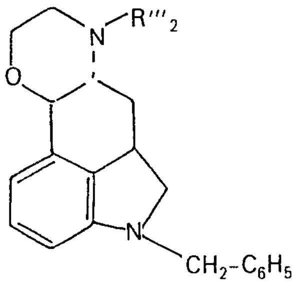

$I'_B$

in which $R'''_2$ has the significance already indicated, corresponding to a product with the formula (I′) in which $R'_2$ has the significance of $R'''_2$, $R_1$ represents a hydrogen atom and R represents a benzyl radical, which product with the formula ($I'_B$), if desired, when $R'''_2$ represents a hydroxyalkyl radical, is treated with a halide agent so as to obtain a product with the formula ($I''_B$):

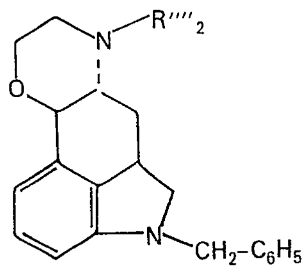

$I''_B$

in which $R''''_2$ represents an alkylhalide radical, corresponding to a product with the formula (I′) in which $R'_2$ has the significance of $R''''_2$, $R_1$ represents a hydrogen atom and R represents a benzyl radical;

b) or, when $R''_2$ represents a hydrogen atom, is made to react with a methylation agent, so as to obtain a product with the formula ($I'_C$):

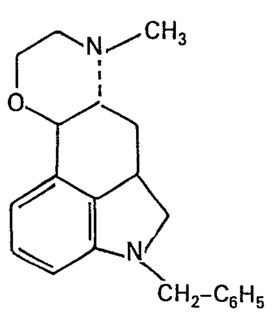

$I'_C$

corresponding to a product with the formula (I′) in which $R'_2$ represents a methyl radical, $R_1$ represents a hydrogen atom and R represents a benzyl radical, which products with the formulae ($I'_A$), ($I'_B$), ($I''_B$) and ($I'_C$), if desired, are hydrogenolized so as to obtain a product with the formula ($I'_D$):

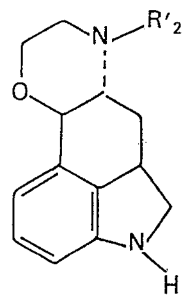

$I'_D$

in which $R'_2$ has the significance already indicated, corresponding to the significance of the substituent of the nitrogen atom in the products with the formulae ($I'_A$), ($I'_B$), ($I''_B$), ($I'_C$), the product with the formula ($I'_D$) corresponding to a product with the formula (I′) in which $R'_2$ has the value indicated above and R and $R_1$ represent a hydrogen atom, which product with the formula ($I'_D$), if desired,

- either is isolated,
- or is made to react with a halide with the formula (VII):

Hal–R′ (VII)

in which Hal represents a chlorine, bromine or iodine atom and R′ represents an alkyl radical containing 1–5 carbon atoms or an aralkyl radical containing 7–12 carbon atoms, possibly substituted, so as to obtain a product with the formula ($I''_D$):

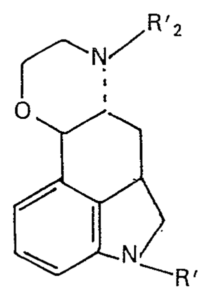

$I''_D$

in which R′ and $R'_2$ have the significance already indicated, corresponding to a product with the formula (I′) in which $R'_2$ has the value indicated above, $R_1$ represents a hydrogen atom and R has the value of R′, then, if desired, the products with the formulae ($I'_A$), ($I'_B$), ($I''_B$), ($I'_C$), ($I'_D$), ($I''_D$) are salified.

7. Process according to Claim 6 characterized in that:

a) the cyclization of the product with the formula (IV) is carried out by means of sodium hydride,

b) the product with the formula (V) is reduced by means of aluminium-lithium hydride,

c) the hydrogenolysis of the products with the formulae ($I'_A$), ($I'_B$), ($I''_B$) and ($I'_C$) is carried out by means of hydrogen in the presence of palladium.

8. Medicaments, characterized in that they are constituted by the derivatives of indolobenzoxazine, as defined by formula (I′) of Claim 1, as well as by their salts of addition with pharmaceutically acceptable acids.

9. Medicaments, characterized in that they are constituted by the derivatives of indolobenzoxazine, as defined in Claim 2 or 3, as well as by their salts of addition with pharmaceutically acceptable acids.

10. Medicaments, characterized in that they are constituted by the derivatives of indolobenzoxazine, as defined in Claim 4, as well as by their salts of addition with pharmaceutically acceptable acids.

11. Medicaments, characterized in that they are constituted by the pharmaceutically acceptable derivatives of indolobenzoxazine, as defined in Claim 5.

12. Pharmaceutical compositions, characterized in that they contain, as active principle, one at least of the medicaments as defined in one of the Claims 8, 9, 10 or 11.

**Claims**

For the contracting State: AT

1. Preparation process for the derivatives of indolobenzoxazine, as well as their salts of addition with mineral or organic acids corresponding to the general formula (I′):

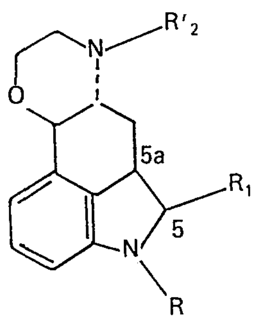

I′

in which R represents a hydrogen atom, an alkyl radical containing 1-5 carbon atoms, or an aralkyl radical containing 7-12 carbon atoms, possibly substituted by chloro, bromo, methyl, ethyl, methoxy, trifluoromethyl or methylthio radicals, $R_1$ represents a hydrogen, chlorine or bromine atom, $R'_2$ represents a hydrogen atom, an alkyl radical containing 1-5 carbon atoms possibly substituted by a halogen atom or by a hydroxy radical, $R'_2$ further represents a cycloalkylalkyl radical containing 4-7 carbon atoms, an alkenyl or alkynyl radical containing 3-7 carbon atoms or an aralkyl radical containing 7-12 carbon atoms, possibly substituted by chloro, bromo, methyl, ethyl, methoxy, trifluoromethyl or methylthio radicals, characterized in that a product with the formula (II):

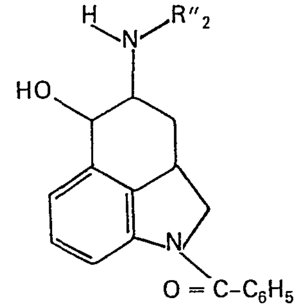

II

in which $R''_2$ represents a hydrogen atom, an alkyl radical containing 1-5 carbon atoms, a cycloalkylalkyl radical containing 4-7 carbon atoms, or an aralkyl radical containing 7-12 carbon atoms, possibly substituted, is reduced so as to obtain a product with the formula (III):

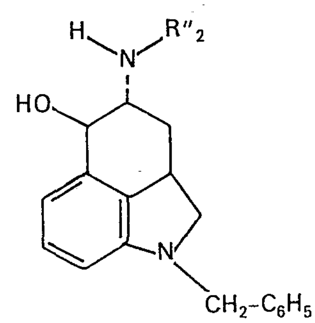

III

in which $R''_2$ has the significance already indicated, which is made to react with chloroacetyl chloride so as to obtain a product with the formula (IV):

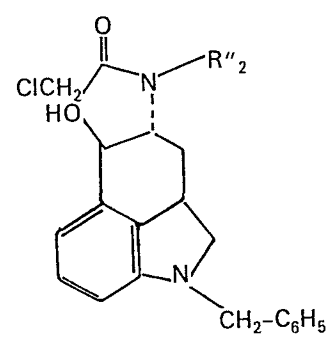

IV

in which $R''_2$ has the significance already indicated, which is cyclized so as to obtain a product with the formula (V):

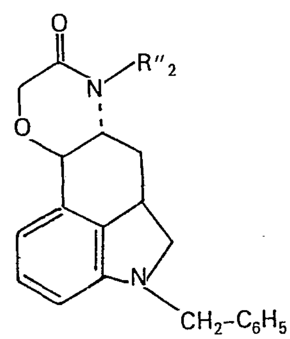

V

in which $R''_2$ has the significance already indicated, which is reduced so as to obtain a product with the formula ($I'_A$):

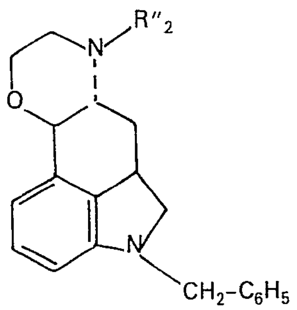

$I'_A$

in which $R''_2$ has the significance already indicated, corresponding to a product with the formula ($I'$), in which $R'_2$ has the significance of $R''_2$, $R_1$ represents a hydrogen atom and R represents a benzyl radical, which product with the formula ($I'_A$), if applicable,

a) either when $R''_2$ represents a hydrogen atom is made to react with a halide with the formula (VI):

Hal-$R'''_2$ (VI)

in which Hal represents a chlorine, bromine or iodine atom, and $R'''_2$ has the significance of $R'_2$ already indicated with the exception of hydrogen and an alkyl halide, so as to obtain a product with the formula ($I'_B$):

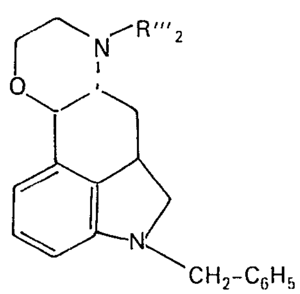

$I'_B$

in which $R'''_2$ has the significance already indicated, corresponding to a product with the formula ($I'$) in which $R'_2$ has the significance of $R'''_2$, $R_1$ represents a hydrogen atom and R represents a benzyl radical, which product with the formula ($I'_B$), if desired, when $R'''_2$ represents a hydroxyalkyl radical, is treated with a halogenation agent so as to obtain a product with the formula ($I''_B$):

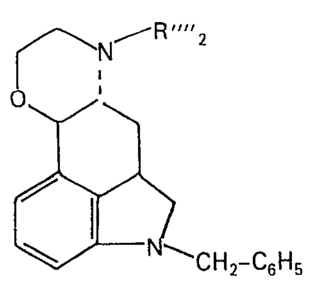

$I''_B$

in which $R''''_2$ represents an alkyl halide radical, corresponding to a product with the formula ($I'$) in which $R_2$ has the significance of $R''''_2$, $R_1$ represents a hydrogen atom and R represents a benzyl radical;

b) or, when $R''_2$ represents a hydrogen atom, is made to act with a methylation agent, so as to obtain a product with the formula ($I'_C$):

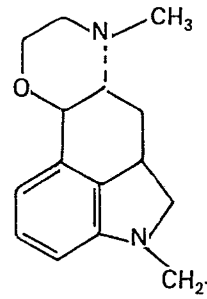

$I'_C$

corresponding to a product with the formula ($I'$) in which $R'_2$ represents a methyl radical, $R_1$ represents a hydrogen atom and R represents a benzyl radical, which products with the formulae ($I'_A$), ($I'_B$), ($I''_B$) and ($I'_C$), if desired are hydrogenolized so as to obtain a product with the formula ($I'_D$):

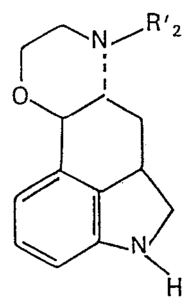

$I'_D$

in which $R'_2$ has the significance already indicated, corresponding to the significance of the substituent of the nitrogen atom in the products with the formulae ($I'_A$), ($I'_B$), ($I''_B$) and ($I'_C$), product with the formula ($I'_D$) corresponding to a product with the formula ($I'$) in which $R'_2$ has the value indicated above and R and $R_1$ each represent a hydrogen atom, which product with the formula ($I'_D$), if desired,

- either is isolated,
- or is made to react with a halide with the formula (VII):

Hal-R' (VII)

in which Hal represents a chlorine, bromine or iodine atom and R' represents an alkyl radical containing 1-5 carbon atoms or an aralkyl radical containing 7-12 carbon atoms, possibly substituted, so as to obtain a product with the formula ($I''_D$):

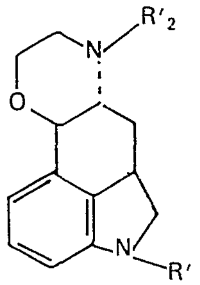

$I''_D$

in which $R'$ and $R'_2$ have the significance already indicated, corresponding to a product with the formula ($I'$) in which $R'_2$ has the value indicated above, $R_1$ represents a hydrogen atom and R has the value of $R'$, then, if desired, the products with the formulae ($I'_A$), ($I'_B$), ($I''_B$), ($I'_C$), ($I'_D$), or ($I''_D$) are salified.

2. Process according to Claim 1, for preparing the derivatives of indolobenzoxazine with the formula ($I'$) as well as their salts corresponding to the general formula (I):

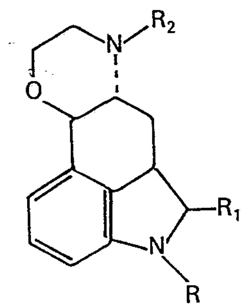

I

in which R and $R_1$ are defined as in Claim 1 and $R_2$ represents a hydrogen atom, an alkyl radical containing 1–5 carbon atoms, a cycloalkylalkyl containing 4–7 carbon atoms, an alkenyl or alkynyl radical containing 3–7 carbon atoms, or an aralkyl radical containing 7–12 carbon atoms, possibly substituted by chloro, bromo, methyl, ethyl, methoxy, trifluoromethyl or methylthio radicals, characterized in that a product with the formula (II):

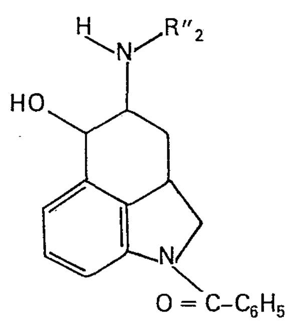

II

in which $R''_2$ represents a hydrogen atom, an alkyl radical containing 1–5 carbon atoms, a cycloalkylalkyl radical containing 4–7 carbon atoms or an aralkyl radical containing 7–12 carbon atoms, possibly substituted, is reduced so as to obtain a product with the formula (III):

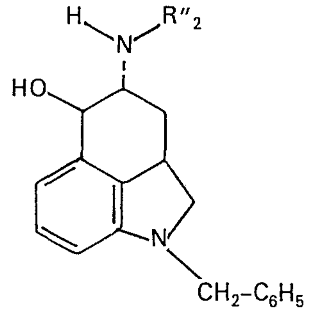

III

in which $R''_2$ has the significance already indicated, which is made to react with chloroacetyl chloride so as to obtain a product with the formula (IV):

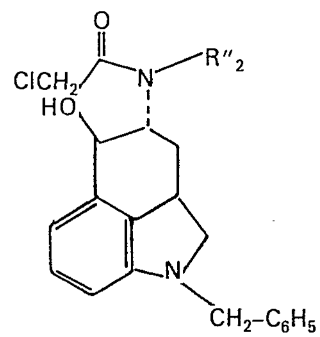

IV

in which $R''_2$ has the significance already indicated, which is cyclized so as to obtain a product with the formula (V):

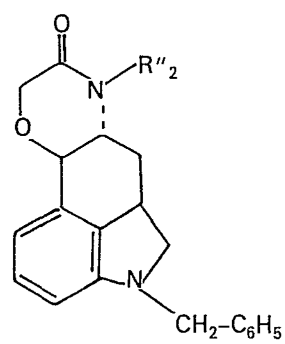

V

in which $R''_2$ has the significance already indicated, which is reduced so as to obtain a product with the formula ($I'_A$):

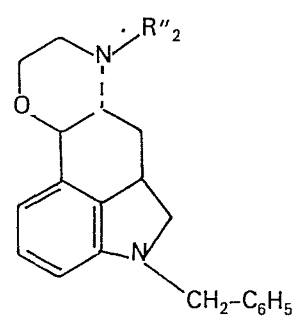

$I'_A$

in which $R''_2$ has the significance already indicated, corresponding to a product with the formula (I'), in which $R'_2$ has the significance of $R''_2$, $R_1$ represents a hydrogen atom and R represents a benzyl radical, which product with the formula ($I'_A$), if applicable,

a) either, when $R''_2$ represents a hydrogen atom, is made to react with a halide with the formula (VI):

Hal-$R'_2$ (VI)

in which Hal represents a chlorine, bromine or iodine atom, and $R'_2$ has the significance of $R_2$ already indicated with the exception of hydrogen, so as to obtain a product with the formula ($I_B$):

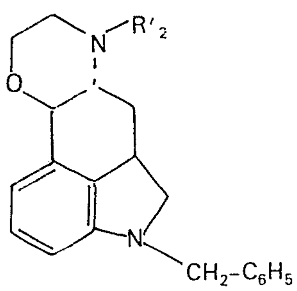

($I_B$)

in which $R'_2$ has the significance already indicated, corresponding to a product with the formula (I) in which $R_2$ has the significance of $R'_2$, $R_1$ represents a hydrogen atom and R represents a benzyl radical,

b) or, when $R''_2$ represents a hydrogen atom, is made to act with a methylation agent, so as to obtain a product with the formula ($I'_C$):

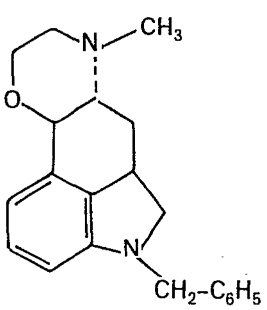

$I'_C$

corresponding to a product with the formula (I) in which $R_2$ represents a methyl radical, $R_1$ represents a hydrogen atom and R represents a benzyl radical, which products with the formulae ($I'_A$), ($I'_B$) and ($I'_C$), if desired, are hydrogenolized so as to obtain a product with the formula ($I_D$):

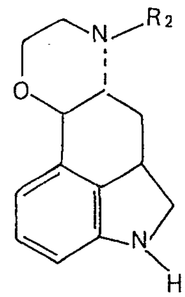

($I_D$)

in which $R_2$ has the significance already indicated, corresponding to the significance of the substituent of the nitrogen atom in the products with the formulae ($I'_A$), ($I_B$) and ($I'_C$), the product with the formulae ($I_D$) corresponding to a product with the formula (I) in which $R_2$ has the value indicated above, R and $R_1$ each represent a hydrogen atom, then, if desired, the products with the formulae ($I'_A$), ($I'_B$), ($I'_C$) or ($I_D$) are salified.

3. Process according to Claim 2, characterized in that:

a) cyclization of the product with the formula (IV) is carried out by means of sodium hydride,

b) the product with the formula (V) is reduced by means of aluminium-lithium hydride,

c) the hydrogenolysis of the products with the formulae ($I'_A$), ($I_B$) and ($I'_C$) is carried out by means of hydrogen in the presence of palladium.

4. Process according to Claim 2, characterized in that a product with the formula (II) in which $R''_2$ represents a hydrogen atom or an alkyl radical containing 1–5 carbon atoms, or a cycloalkylalkyl radical containing 4–7 carbon atoms, or a reagent with the formula (VI) in which $R'_2$ represents an alkyl radical containing 1–5 carbon atoms or a cycloalkylalkyl radical containing 4–7 carbon atoms is used at the start.

5. Process according to Claim 2, characterized in that a product with the formula (II) in which $R''_2$ represents a hydrogen atom or an alkyl radical containing 1–5 carbon atoms, or a reagent with the formula (VI) in which $R'_2$ represents an alkyl radical containing 1–5 carbon atoms is used at the start.

6. Process according to any one of Claims 1–5, characterized in that 5,5a, 6,6a (RS), 7, 8, 9, 10a (RS)- octahydro-7- propyl -4H-indolo-[3,4-g.h]-[1,4]benzoxazine as well as its salts of addition with mineral or organic acids is prepared.